# FASCICULE DE BREVET EUROPEEN

(11) **EP 4 262 999 B1**
(45) Date de publication et mention de la délivrance du brevet: **12.02.2025**
(21) Numéro de dépôt: 21854671.1
(22) Date de dépôt: 14.12.2021
(51) Int. Cl.: A61Q 19/00, A61Q 19/08, A61K 8/99, A61P 17/00

(54) **UTILISATION COSMÉTIQUE, NUTRACEUTIQUE OU DERMATOLOGIQUE D'UNE SOUCHE DE LACTOBACILLUS CRISPATUS ET/OU D'UNE COMPOSITION LA COMPRENANT**
KOSMETISCHE, NUTRAZEUTISCHE ODER DERMATOLOGISCHE VERWENDUNG EINES LACTOBACILLUS CRISPRATUS-STAMMS UND/ODER EINER ZUSAMMENSETZUNG DAMIT
COSMETIC, NUTRACEUTICAL OR DERMATOLOGICAL USE OF A LACTOBACILLUS CRISPATUS STRAIN AND/OR OF A COMPOSITION COMPRISING SAME

(30) Priorité: 15.12.2020 FR 2013222
(43) Date de publication de la demande: 25.10.2023
(73) Titulaire: BASF Beauty Care Solutions France SAS, 69007 Lyon (FR); BASF Corporation, Florham Park, NJ 07932 (US)
(72) Inventeur: ABRAHAM, Neethu, Ardsley, NY 10502 (US); ANDRE, Valérie, 69007 Lyon (FR); DEL BENE, Nicolas Stefano, Florham Park, NJ 07932 (US); GARLET, Allison, Tarrytown, NY 10591-9005 (US); GAULT, Manon, 69007 Lyon (FR); LEOTY-OKOMBI, Sabrina, 69007 Lyon (FR)
(74) Mandataire: Cabinet Becker et Associés
(86) Numéro de dépôt international: PCT/FR2021/052325
(87) Numéro de publication internationale: WO 2022/129775

(56) Documents cités:
- EP-A1- 3 040 413
- EP-A2- 1 778 258
- EP-B1- 0 941 056
- WO-A1-2019/111189
- WO-A2-2009/031106
- US-A1- 2015 093 462

## Description

La présente invention concerne l'utilisation cosmétique, nutraceutique et / ou pharmaceutique, notamment dermatologique, d'une souche de *Lactobacillus crispatus* et / ou d'une composition la comprenant pour maintenir et / ou augmenter l'expression des molécules de la matrice extracellulaire de la peau et / ou des muqueuses et notamment pour maintenir et / ou augmenter les propriétés biomécaniques de la peau saine et / ou des muqueuses saines, préférentiellement la fermeté et / ou l'élasticité et / ou la densité de la peau saine et / ou des muqueuses saines, encore préférentiellement la fermeté et / ou l'élasticité de la peau saine et / ou des muqueuses saines.

La structure et les propriétés de la peau changent sous l'effet de processus biologiques, physiques et biomécaniques complexes, entrainant une diminution d'élasticité, de fermeté, de densité du derme, notamment sous l'effet de la modification des molécules de la matrice extracellulaire.

La matrice extracellulaire de la peau et des muqueuses, dite MEC, est composée de l'association de trois types de molécules :
- des fibres : collagène et élastine,
- des glycoprotéines : moins abondantes que les fibres mais ont un rôle plus important dans l'adhérence cellulaire, comme la fibronectine et la laminine,
- des polysaccharides très hydratés constituant un gel de remplissage de la matrice. Cette matrice joue un rôle prépondérant dans le maintien de la structure et les propriétés de la peau, notamment l'élasticité, la fermeté ainsi que la densité du derme.

Ces molécules de la MEC étant notamment synthétisées par les fibroblastes et les kératinocytes, le renouvellement de ces derniers est primordial pour le maintien des propriétés biomécaniques de la peau.

L'élastine est une protéine composant les fibres élastiques en s'associant avec d'autres molécules comme les fibrillines et les MAGP (Microfibrillar Associated Glycoproteins).

L'élastine est synthétisée sous la forme de tropoélastine soluble qui acquiert ses propriétés physico-chimiques (insolubilité, élasticité) après sa réticulation intra- et intermoléculaire par une lysyl oxydase (LOX) et son dépôt sur les microfibrilles. Dans la peau, les fibres élastiques fonctionnelles sont formées par les fibroblastes du derme, mais certains composants nécessaires à l'élaboration des fibres élastiques ont aussi été retrouvés dans les cellules de l'épiderme.

Le taux de renouvellement des fibres élastiques est très faible dans la vie adulte, bien que le taux global d'élastine de la peau puisse augmenter. Chez le nouveau-né, les microfibrilles ne sont pas toutes complètement couvertes d'élastine, et le deviennent vers la puberté. Dès 40 ans, on voit apparaître sur les fibres des inclusions, plus fréquentes chez les femmes, puis la fragmentation des fibres élastiques et leur disparition sous la jonction dermo-épidermique (JDE). Ce fractionnement et / ou cette disparition sous la JDE se traduisent par la diminution d'élasticité de la peau et la formation de rides. La synthèse de fibres élastiques non fonctionnelles est observée lors du photo-vieillissement, mais cette augmentation s'accompagne de la diminution accélérée des fibres élastiques sous la JDE.

Le collagène est une protéine constitutive de la matrice extra-cellulaire (MEC) présente en grande quantité dans les tissus des vertébrés. Il s'agit d'une large famille comprenant 29 types différents.

Parmi les différents types de collagène, on retrouve en particulier le collagène de type I dans de nombreux tissus humains tels que tendons, ligaments, cornée et peau, localisé plus précisément au niveau du derme. Le collagène de type I est le collagène majoritaire de la peau.

Le collagène fibrillaire est formé à partir des fibres de procollagène, ensuite assemblées en réseau, puis stabilisées par réticulation. C'est le collagène qui confère aux tissus leur résistance mécanique, et par voie de conséquence, participe au maintien de leur fermeté.

Au cours du vieillissement de la peau, le taux de collagène synthétisé diminue globalement et leur dégradation augmente ce qui entraîne une diminution de fermeté.

Ce phénomène est d'autant plus prononcé que la peau est entre autres soumise aux UV. On parle de vieillissement photobiologique.

Les molécules de la matrice extracellulaire sont donc l'objet de nombreuses études et innovations dans le domaine de la cosmétique pour la mise au point et l'amélioration d'actifs cosmétiques visant à maintenir les propriétés biomécaniques de la peau.

Depuis quelques années des techniques de séquençage génomique poussées ont mis en évidence que sur la peau vivent des millions de microorganismes (bactéries, virus, champignons) qui composent le microbiote cutané encore appelée flore cutanée. Ces microorganismes (ce microbiote) pour la plupart sont commensaux et bénéfiques à la peau. Ils cohabitent et interagissent avec les cellules cutanées et pour certains ont développé une symbiose avec la peau. Ils participent à la défense de la peau en synthétisant ou en stimulant la synthèse de molécules de défenses de la peau. Ils participent à la réparation de la peau en cas de lésion et luttent contre l'inflammation. Ces microorganismes commensaux bénéfiques sécrètent des molécules et des métabolites qui participent à l'homéostasie et à l'équilibre de la peau.

La composition et la qualité du microbiote de la peau évolue avec le vieillissement. De manière surprenante, la demanderesse a démontré qu'avec l'âge certaines bactéries étaient moins présentes et moins abondantes sur la peau. C'est notamment le cas de la bactérie *Lactobacillus crispatus.*

La plupart des solutions existant sur le marché traitent des conséquences visibles du vieillissement et ne prennent pas en compte le microbiote cutané dont certaines espèces vivent en symbiose avec les cellules cutanées et participent à son équilibre et à son aspect.

La présente invention a l'avantage d'être plus complète dans le sens où elle rééquilibre le microbiote d'une peau âgée en le complétant avec des bactéries de la flore cutanée qui diminuent avec le temps. Ces bactéries à leur tour participent au rajeunissement et à une protection durable de la peau contre les agressions extérieures, en relançant des processus biologiques qui diminuent avec l'âge.

Des bactéries probiotiques et leurs dérivés sont déjà utilisés pour traiter les problèmes de vieillissement cutané, mais dans la plupart des cas les bactéries utilisées ne proviennent pas de la peau. La bactérie selon la présente invention présente l'avantage d'être naturellement présente sur la peau jeune.

La demande WO2009031106 divulgue l'utilisation d'une combinaison d'hespéridine et d'un probiotique pour prévenir la réduction ou renforcer la fonction barrière de la peau. La demande ne divulgue pas l'utilisation de souche de *Lactobacillus crispatus* pour maintenir et / ou augmenter l'expression des molécules de la matrice extracellulaire de la peau et / ou des muqueuses.

La demande WO2019111189 divulgue l'utilisation comprenant un probiotique du genre Lactobacillus pour la prévention des dommages induits par les radiations UV. La demande ne divulgue pas l'utilisation de souche de *Lactobacillus crispatus* pour maintenir et / ou augmenter l'expression des molécules de la matrice extracellulaire de la peau et / ou des muqueuses.

Ainsi, à la connaissance de la demanderesse, aucun art antérieur ne divulgue ni ne suggère l'utilisation cosmétique, nutraceutique et / ou pharmaceutique, notamment dermatologique d'une souche de *Lactobacillus crispatus* et / ou d'une composition la comprenant pour maintenir et / ou augmenter l'expression des molécules de la matrice extracellulaire de la peau et / ou des muqueuses, ni pour maintenir et / ou augmenter les propriétés biomécaniques de la peau et / ou des muqueuses, ni pour maintenir et / ou augmenter l'expression des collagènes de la peau et / ou des muqueuses et / ou des fibres élastiques de la peau et / ou des muqueuses.

La présente invention a pour premier objet l'utilisation cosmétique et / ou nutraceutique d'une souche de *Lactobacillus crispatus* pour maintenir et / ou augmenter l'expression des molécules de la matrice extracellulaire de la peau saine et / ou des muqueuses saines.

Elle a également pour objet un procédé de soin cosmétique caractérisé en ce qu'il comprend l'application par voie topique sur au moins une zone de peau saine et / ou de muqueuse saine d'une souche de *Lactobacillus crispatus* selon l'invention, ou d'une composition cosmétique la comprenant, pour maintenir et / ou augmenter l'expression des molécules de la matrice extracellulaire de la peau saine et / ou des muqueuses saines, préférentiellement pour maintenir et / ou augmenter l'expression des collagènes de la peau saine et / ou des muqueuses saines et / ou des fibres élastiques de la peau saine et / ou des muqueuses saines, et / ou pour maintenir et / ou augmenter les propriétés biomécaniques de la peau saine et / ou des muqueuses saines, notamment la fermeté et / ou l'élasticité et / ou la densité, préférentiellement la fermeté.

L'invention a encore pour objet une souche de *Lactobacillus crispatus* selon l'invention, ou d'une composition dermatologique la comprenant, pour le traitement et / ou la prévention des pathologies de la peau et / ou des muqueuses impliquant une diminution de la quantité et/ou de l'expression des molécules de la matrice extracellulaire de la peau et / ou des muqueuses, notamment une diminution de la quantité et/ou de l'expression des collagènes de la peau et / ou des muqueuses et / ou des fibres élastiques de la peau et / ou des muqueuses, et / ou une diminution des propriétés biomécaniques de la peau et / ou des muqueuses, notamment une diminution de la fermeté de la peau et / ou des muqueuses et / ou de l'élasticité et / ou de la densité.

Elle concerne également une souche de *Lactobacillus crispatus* déposée suivant le traité de Budapest auprès de l'Institut Pasteur (28 rue du Docteur Roux, F-75024 Paris cedex 15) le 09/09/2020 sous la désignation CNCM I-5579 ou d'une composition cosmétique la comprenant, pour maintenir et / ou augmenter l'expression des molécules de la matrice extracellulaire de la peau saine et / ou des muqueuses saines, notamment pour maintenir et / ou augmenter l'expression des collagènes de la peau saine et / ou des muqueuses saines et / ou des fibres élastiques de la peau saine et / ou des muqueuses saines, et / ou pour maintenir et / ou augmenter les propriétés biomécaniques de la peau saine et / ou des muqueuses saines, notamment la fermeté et / ou l'élasticité et / ou la densité, préférentiellement la fermeté.

Elle concerne enfin une souche de *Lactobacillus crispatus* déposée suivant le traité de Budapest auprès de l'Institut Pasteur (28 rue du Docteur Roux, F-75024 Paris cedex 15) le 09/09/2020 sous la désignation CNCM I-5579 ou d'une composition dermatologique la comprenant, pour le traitement et / ou la prévention des pathologies de la peau et / ou des muqueuses impliquant une diminution de la quantité et/ou de l'expression des molécules de la matrice extracellulaire de la peau et / ou des muqueuses, notamment une diminution de la quantité et/ou de l'expression des collagènes et / ou des fibres élastiques de la peau et / ou des muqueuses, et / ou une diminution de la quantité et/ou des propriétés biomécaniques de la peau et / ou des muqueuses, notamment une diminution de la fermeté de la peau et / ou des muqueuses et / ou une diminution de l'élasticité de la peau et / ou des muqueuses et / ou une diminution de la densité de la peau et / ou des muqueuses.

Au sens de la présente invention, on entend par « utilisation et / ou composition cosmétique », une utilisation et / ou composition non pharmaceutique, c'est-à-dire qui n'est pas destinée à un usage thérapeutique et est appliquée sur une partie du corps dite saine, en particulier sur une zone de la peau et / ou des muqueuses dite saine. Au sens de la présente invention, on entend par « utilisation nutraceutique et / ou composition nutraceutique » une utilisation et / ou une composition destinée à être utilisée par voie orale non pharmaceutique, c'est-à-dire qui ne nécessite pas de traitement thérapeutique.

Au sens de la présente invention on entend par « peau saine », « muqueuse saine » une zone de peau, de muqueuse sur laquelle est appliquée la souche selon l'invention et dite « non pathologique » par un dermatologue, c'est-à-dire ne présentant pas d'infection, de cicatrice, de maladie ou d'affection cutanée telle que candidose, impétigo, psoriasis, eczéma, acné ou dermatite, ou de plaies ou de blessures.

Selon l'invention, on désigne par « muqueuse(s) », la muqueuse oculaire, la muqueuse nasale, la muqueuse auriculaire, la muqueuse uro-génitale et / ou la muqueuse buccale, notamment buccale, labiale et / ou gingivale, préférentiellement, la muqueuse oculaire et / ou buccale, et encore préférentiellement, la muqueuse labiale et / ou oculaire.

Avantageusement, elle ne comprend pas la muqueuse vaginale.

Au sens de la présente invention, on entend par « maintenir et / ou augmenter l'expression des molécules de la matrice extracellulaire », empêcher une diminution et / ou augmenter le niveau d'expression génique, c'est-à-dire des ARNm (ARN messagers), et / ou de la synthèse protéique des molécules de la matrice extracellulaire de la peau saine et / ou des muqueuses saines, traitées par la souche selon l'invention, par rapport au niveau d'expression génique et / ou de synthèse protéique détecté en l'absence de la souche selon l'invention. Préférentiellement, il s'agit du maintien et / ou de l'augmentation de l'expression protéique des molécules de la matrice extracellulaire.

Selon l'invention, on entend par « molécules de la MEC », les molécules organiques qui constituent et/ou sont contenues dans la matrice extracellulaire et qui sont synthétisées par des cellules, d'origine humaines ou animales.

Il s'agit notamment des protéines constitutives de la MEC, en particulier sélectionnées parmi :
- La famille des collagènes de la MEC,
- La famille des fibres élastiques de la MEC : l'élastine, la tropoélastine, les protéines associées à l'élastine notamment la fibrilline 1, les lysyl oxydases, notamment LOX et LOXL, l'EBP (Elastine Binding Protein), les fibullines 3 et 5, les Emilin 1 et 2.

Il s'agit notamment des protéoglycanes constitutifs de la MEC, en particulier les protéoglycanes sécrétés choisis parmi le Perlecan, le versican, les protéoglycanes riches en leucine (SLRPs) notamment la decorine, le biglycan et le lumican.

Il s'agit également des glycoprotéines constitutifs de la MEC, notamment la fibronectine, la laminine, la SPARC (Secreted Protein Rich in Cystein), la tenascine . Il s'agit en outre des glycosaminoglycans (GAG) constitutifs de la MEC, en particulier l'acide hyaluronique, heparane sulfate, dermatan sulfate, chondroitine sulfate.

Il s'agit également des facteurs de croissance de la MEC qui sont des protéines contenues dans la MEC, notamment le VEGF, le PDGF, le HGF, les FGF, et en particulier FGF2 et FGF 7.

Les « molécules de la MEC » désignent les molécules dans la MEC ou dans d'autres compartiments notamment intracellulaire et extracellulaire dans le milieu de culture, éventuellement sous forme de précurseurs.

Selon l'invention, on entend par « précurseur de molécule de la MEC » une forme native et/ou intermédiaire de la molécule de la MEC, synthétisée par la cellule, avant de constituer ou être contenue dans la MEC. Ces formes subissent des phénomènes de maturation avant d'être dans la MEC et sont ainsi souvent désignées par les préfixes « Pro-» ou «Tropo- ». Il s'agit par exemple les procollagènes, les tropocollagènes, la proélastine, la tropoélastine.

Préférentiellement, les molécules de la matrice extracellulaire sont choisies parmi les collagènes de la peau saine et / ou des muqueuses saines, et / ou les fibres élastiques de la peau saine et / ou des muqueuses saines.

Au sens de la présente invention, on entend par « maintenir et / ou augmenter l'expression des collagènes de la peau saine et / ou des muqueuses saines et / ou des fibres élastiques de la peau saine et / ou des muqueuses saines» empêcher une diminution et / ou augmenter le niveau d'expression génique, c'est-à-dire l'expression des ARNm (ARN messagers), et / ou de la synthèse protéique des collagènes de la peau saine et / ou des muqueuses saines et / ou des fibres élastiques de la peau saine et / ou des muqueuses saines traitées par la souche selon l'invention, par rapport à l'expression génique et / ou de la synthèse protéique mesurée en l'absence de la souche selon l'invention.

En particulier, il s'agit d'une augmentation du niveau d'expression génique et / ou de la synthèse protéique des collagènes et / ou des fibres élastiques d'au moins 20 %, préférentiellement d'au moins 40 % et encore plus préférentiellement d'au moins 50 %, par rapport au niveau d'expression génique et / ou protéique des collagènes et / ou des fibres élastiques mesuré en l'absence de la souche de *Lactobacillus crispatus* selon l'invention.

Dans un mode préférentiel de l'invention, il s'agit d'une augmentation du niveau d'expression protéique de la fibuline-5 et / ou de l'emilin-1 d'au moins 20 %, préférentiellement d'au moins 40 % et encore plus préférentiellement d'au moins 50 % par rapport au niveau d'expression protéique mesuré dans des cellules de fibroblastes dermiques cultivées *in vitro* en l'absence de la souche, comme par exemple tel que décrit dans l'exemple 3.

Avantageusement, la mesure de l'augmentation de l'expression protéique de fibuline-5 et/ou d'emilin-1 est effectuée par mesure *in vitro,* préférentiellement après analyse par électrophorèse capillaire, par exemple selon la méthode telle que présentée en exemple 3.

Au sens de la présente invention, on entend par « analyse par électrophorèse capillaire », la technique consistant à fixer un anticorps spécifique de la protéine étudiée fibuline-5 et / ou emilin-1, sur ladite protéine, conjugué à une peroxydase et un substrat chemiluminescent, en vue de la détection de la protéine par mesure du signal chemiluminescent.

Au sens de la présente invention, on entend par « collagène », les protéines de collagène de type I, III, IV, V, VI, VII, XII, XIII, XIV, XVI, XVII, XXIV, XXIX, présentes dans la peau saine et / ou les muqueuses saines. Selon un mode avantageux, les collagènes sont choisis parmi les collagènes de type I, III, IV, V et / ou XIV, préférentiellement les collagènes de type I, IV et / ou V, encore préférentiellement le collagène de type I.

Au sens de la présente invention, on entend par protéine de « collagène de type I », la protéine de collagène présente dans la peau, la cornée, les tendons, les ligaments, les os, encore préférentiellement dans la peau. Ainsi, dans un mode préférentiel de l'invention, la souche de *Lactobacillus crispatus* selon l'invention est donc utilisée pour maintenir et / ou augmenter l'expression protéique du collagène de type I au niveau de la peau saine et / ou des muqueuses saines.

Selon un mode avantageux de l'invention, il s'agit d'une augmentation par rapport au niveau d'expression génique et / ou protéique du collagène mesuré dans des cellules de fibroblastes dermiques cultivées *in vitro* en l'absence de la souche de *Lactobacillus crispatus* selon l'invention.

Dans un mode préférentiel de l'invention, il s'agit d'une augmentation du niveau d'expression protéique du collagène de type I d'au moins 10 %, préférentiellement d'au moins 50 % et encore plus préférentiellement d'au moins 20 % par rapport au niveau d'expression protéique mesuré dans des cellules de fibroblastes dermiques cultivées *in vitro* en l'absence de la souche selon le protocole tel que décrit par exemple dans l'exemple 2.

Avantageusement, la mesure de l'augmentation de l'expression protéique est effectuée par mesure *in vitro,* préférentiellement après dosage immunoenzymatique ou ELISA par exemple selon la méthode telle que présentée en exemple 2.

Au sens de la présente invention, on entend par « dosage immunoenzymatique », la technique consistant à fixer un anticorps spécifique d'une protéine donnée de collagène, préférentiellement la protéine de collagène de type I, sur ladite protéine, en vue d'une révélation par mesure de fluorescence. Avantageusement, la révélation se fait par fluorescence.

Selon un autre mode avantageux, la mesure de l'augmentation de l'expression génique est effectuée par mesure *in vitro,* préférentiellement après RT-qPCR.

Dans une forme d'exécution préférée, la souche selon l'invention est utilisée pour maintenir et / ou augmenter les propriétés biomécaniques de la peau saine et / ou des muqueuses saines, préférentiellement la fermeté et / ou l'élasticité et / ou la densité de la peau saine et / ou des muqueuses saines, encore préférentiellement la fermeté de la peau saine et / ou des muqueuses saines, en particulier du derme sain et / ou l'élasticité de la peau saine et / ou des muqueuses saines, en particulier du derme sain.

Au sens de la présente invention on entend par « propriétés biomécaniques de la peau saine et / ou des muqueuses saines » la fermeté et / ou l'élasticité et / ou la densité et / ou la résistance à la compression et / ou la résistance à l'étirement et / ou la flexibilité et / ou l'extensibilité et / ou la capacité à résister à la déformation de la peau saine et / ou des muqueuses saines. Préférentiellement il s'agit de la fermeté et / ou l'élasticité et / ou la densité de la peau saine et / ou des muqueuses saines, en particulier du derme sain, encore préférentiellement il s'agit de la fermeté de la peau saine et / ou des muqueuses saines, en particulier du derme sain et / ou de l'élasticité de la peau saine et / ou des muqueuses saines, en particulier du derme sain.

Les propriétés biomécaniques de la peau saine et / ou des muqueuses saines peuvent être étudiées par les techniques de mesure connues par l'homme du métier notamment par traction, par torsion, succion, indentation, levatométrie, ballistométrie, par scanner ultrason à haute résolution ou élastographie, préférentiellement par indentation, par scanner ultrason à haute résolution.

Au sens de la présente invention, on entend par « maintenir et / ou augmenter les propriétés biomécaniques de la peau saine et / ou des muqueuses saines» empêcher une diminution et / ou augmenter les propriétés biomécaniques de la peau saine et / ou des muqueuses saines, préférentiellement la fermeté de la peau saine et / ou des muqueuses saines, en particulier du derme sain, et / ou l'élasticité de la peau saine et / ou des muqueuses saines, en particulier du derme sain, et / ou la densité de la peau saine et / ou des muqueuses saines, en particulier du derme sain, traité par la souche selon l'invention par rapport aux propriétés biomécaniques de la peau saine et / ou des muqueuses saines mesurées en l'absence de la souche selon l'invention.

Préférentiellement, il s'agit d'une augmentation d'au moins 5%, préférentiellement 10% des propriétés biomécaniques de la peau saine et / ou des muqueuses saines traitées par la souche de *Lactobacillus crispatus* selon l'invention par rapport aux propriétés biomécaniques de la peau saine et / ou des muqueuses saines mesurées en l'absence de la souche de *Lactobacillus crispatus* selon l'invention.

Au sens de la présente invention, on entend d'un point de vue cosmétique par « maintenir et / ou augmenter la fermeté de la peau saine et / ou des muqueuses saines, en particulier du derme sain », empêcher la diminution et / ou augmenter à des fins esthétiques, la fermeté de la peau saine et / ou des muqueuses saines notamment celles qui ont perdu de la fermeté notamment sous l'effet de facteurs intrinsèques. Les facteurs intrinsèques peuvent être le vieillissement tissulaire de la peau et / ou des muqueuses, c'est-à-dire le vieillissement chrono-induit, que l'on retrouve par exemple chez les peaux dites matures c'est-à-dire les peaux de personnes de plus de 40 ans. Il peut s'agir du stress cellulaire, des variations physiologiques non pathologiques telles qu'un changement de régime alimentaire, des variations hormonales, en particulier durant la puberté, la grossesse, la ménopause et / ou l'andropause. Cette diminution de fermeté peut également apparaitre sous l'effet de facteurs extrinsèques, tels que les agents agressifs de l'environnement comme par exemple la pollution, les fumées, le tabac, les toxines, ou les agressions climatiques et / ou mécaniques.

Selon un mode préférentiel, la diminution de fermeté n'est pas induite par les radiations UV.

Préférentiellement, il s'agit d'une augmentation de la fermeté au niveau de la peau saine et / ou des muqueuses saines, en particulier du derme sain, d'au moins 1%, préférentiellement d'au moins 3%, encore avantageusement d'au moins 5% en présence d'une souche de *Lactobacillus crispatus* selon l'invention. Dans un mode de réalisation avantageux de l'invention, il s'agit d'une augmentation mesurée in *vivo,* préférentiellement sur la peau du visage humain.

Au sens de la présente invention, on entend d'un point de vue cosmétique par « maintenir et / ou augmenter l'élasticité de la peau saine et / ou des muqueuses saines, en particulier du derme sain », empêcher la diminution et / ou entrainer l'augmentation à des fins esthétiques, de l'élasticité de la peau saine et / ou des muqueuses saines qui ont perdu de l'élasticité notamment sous l'effet de facteurs intrinsèques, tels que le vieillissement tissulaire de la peau et / ou des muqueuses, c'est-à-dire le vieillissement chrono-induit, que l'on retrouve par exemple chez les peaux dites matures c'est-à-dire les peaux de personnes de plus de 40 ans, en particulier de plus de 50 ans, le stress cellulaire, les variations physiologiques non pathologiques telles que un changement de régime alimentaire, les variations hormonales, en particulier durant la puberté, la grossesse, la ménopause et / ou l'andropause. Cette diminution d'élasticité peut également apparaitre sous l'effet de facteurs extrinsèques, tels que les agents agressifs de l'environnement, comme par exemple la pollution, les fumées, le tabac, les toxines, ou les agressions climatiques et / ou mécaniques.

Selon un mode préférentiel, la diminution d'élasticité n'est pas induite par les radiations UV.

On entend en outre par « augmenter l'élasticité » de la peau et / ou des muqueuses une augmentation de l'élasticité mesurée *in vivo,* d'au moins 2%, avantageusement d'au moins 4%, encore avantageusement d'au moins 6% en présence de la souche selon l'invention par rapport à l'élasticité détectée en l'absence de la souche. Dans un mode de réalisation préférentiel de l'invention, cette mesure est effectuée sur la peau du visage humain. Dans un mode préférentiel, cette augmentation est évaluée après au moins 10, préférentiellement 28, encore préférentiellement 30 et avantageusement au moins 56 jours de traitement en présence de la souche selon l'invention.

De manière encore avantageuse, l'élasticité est évaluée par la mesure de ses différentes composantes de l'élasticité, notamment l'élasticité nette, la récupération élastique et/ou l'élasticité globale, notamment comme décrit dans l'exemple 4.

Au sens de la présente invention, on entend d'un point de vue cosmétique par « maintenir et / ou augmenter la densité de la peau saine et / ou des muqueuses saines, en particulier du derme sain », empêcher la diminution et / ou augmenter à des fins esthétiques, la densité de la peau saine et / ou des muqueuses saines notamment celles qui ont perdu de la densité notamment sous l'effet de facteurs intrinsèques. Les facteurs intrinsèques peuvent être le vieillissement tissulaire de la peau et / ou des muqueuses, c'est-à-dire le vieillissement chrono-induit, que l'on retrouve par exemple chez les peaux dites matures c'est-à-dire les peaux de personnes de plus de 40 ans. Il peut s'agir du stress cellulaire, des variations physiologiques non pathologiques telles qu'un changement de régime alimentaire, des variations hormonales, en particulier durant la puberté, la grossesse, la ménopause et / ou l'andropause. Cette diminution de densité peut également apparaitre sous l'effet de facteurs extrinsèques, tels que les agents agressifs de l'environnement comme par exemple la pollution, les fumées, le tabac, les toxines, ou les agressions climatiques et / ou mécaniques.

Selon un mode préférentiel, la diminution de densité n'est pas induite par les radiations UV.

Préférentiellement, il s'agit d'une augmentation de la densité au niveau de la peau saine et / ou des muqueuses saines, en particulier du derme sain, d'au moins 1%, préférentiellement d'au moins 3%, encore avantageusement d'au moins 5% en présence d'une souche de *Lactobacillus crispatus* selon l'invention. Dans un mode de réalisation avantageux de l'invention, il s'agit d'une augmentation mesurée *in vivo,* préférentiellement sur la peau du visage humain.

La mesure *in vivo* de la fermeté et / ou de l'élasticité et / ou de la densité pourra être effectuée selon les méthodes classiques connues de l'homme de l'art, notamment par mesure à l'aide d'un cutomètre, d'un Tonoderm^{™}, d'un scanner à ultrason, ou encore d'un DynaSKIN^{®} associé à un dermaTOP.

Selon la présente invention, la souche de *Lactobacillus crispatus* peut être utilisée sous forme entière, notamment viable et / ou inactivée, notamment morte, et / ou sous forme de lysat, et / ou sous forme de l'une ou plusieurs de ses fractions, et / ou sous forme d'un ou plusieurs de ses métabolites, préférentiellement de son sécrétome. Pour chacune de ses formes, la souche selon l'invention peut être isolée ou associée à son milieu de fermentation et / ou de culture.

Préférentiellement, la souche selon l'invention est associée à son milieu de fermentation et / ou de culture.

Au sens de la présente invention, on entend par « isolée », non mélangée à un ou des composé(s) susceptible(s) de lui être associé(s) dans son milieu de fermentation et / ou de croissance.

Au sens de la présente invention, on entend par « forme entière » sa forme native, une forme dans laquelle l'enveloppe bactérienne est intacte, par opposition à une forme lysée. Lorsqu'elle est utilisée sous forme entière, la souche *Lactobacillus crispatus* peut être viable et / ou inactivée et / ou morte.

Au sens de la présente invention, on entend par « viable » une souche de *Lactobacillus crispatus* selon la présente invention capable de former des colonies en culture.

La production de la souche de *Lactobacillus crispatus* sous forme entière viable pourra être effectuée selon toute méthode classiquement connue de l'homme du métier. Avantageusement, elle sera effectuée selon le protocole tel que décrit par exemple dans l'exemple 1.a ou 1.f.

Au sens de l'invention, on entend par "inactivée" une souche de *Lactobacillus crispatus* selon la présente invention qui n'est plus capable, temporairement ou définitivement, de former des colonies en culture.

L'inactivation de la souche de *Lactobacillus crispatus* pourra être effectuée selon toute méthode classiquement connue de l'homme du métier. Elle peut notamment être inactivée par irradiation, traitement thermique ou, sous certaines conditions, par lyophilisation, par traitement haute pression ou par extrusion avantageusement, elle sera effectuée par traitement thermique, encore avantageusement selon le protocole tel que décrit par exemple dans l'exemple 1.b.

L'inactivation thermique peut être réalisée en incubant la souche de *Lactobacillus crispatus* pendant une période de temps donnée, avantageusement d'environ 10 s à 90 min, préférentiellement d'environ 15 minutes à une heure, et à une température avantageusement d'environ 60°C à 150 °C. Préférentiellement, elle sera incubée pendant environ 30 minutes à environ 80°C.

Au sens de l'invention, on entend par "morte", une souche de *Lactobacillus crispatus* selon la présente invention qui n'est plus capable, définitivement, de former des colonies en culture.

Au sens de l'invention, on entend par "lysat", un matériau obtenu à l'issue de la destruction ou dissolution de cellules biologiques par un phénomène dit de lyse cellulaire provoquant ainsi la libération des constituants biologiques intracellulaires naturellement contenus dans les cellules du microorganisme considéré et de fragments de composants de membrane cellulaire. Le lysat mis en œuvre est donc formé en l'ensemble les constituants biologiques intracellulaires et des constituants des parois et membranes cellulaires de la souche de *Lactobacillus crispatus* selon l'invention.

Le lysat pourra être obtenu par toute méthode classiquement connue de l'homme du métier. Elle pourra notamment être obtenue par un choc osmotique, un choc thermique, par ultrasons, par lyse enzymatique, par tyndallisation, ou encore sous contrainte mécanique de type centrifugation, ou par augmentation de la pression ou des combinaisons de ces différentes technologies.

Préférentiellement, le lysat sera obtenu par combinaison d'action mécanique et d'augmentation de la pression. Encore préférentiellement, le lysat sera obtenu selon le protocole tel que décrit par exemple dans l'exemple 1.b.

Dans un mode de réalisation préférentiel de l'invention, le lysat sera associé au milieu de culture et / ou de fermentation de la bactérie.

Au sens de la présente invention, on entend par « fractions », un fragment de la souche de *Lactobacillus crispatus* selon la présente invention doté d'une efficacité pour maintenir et / ou augmenter l'expression des molécules de la matrice extracellulaire de la peau saine et / ou des muqueuses saines, et / ou pour maintenir et / ou augmenter les propriétés biomécaniques de la peau saine et / ou des muqueuses saines. Cette fraction correspond à une partie non totale des constituants biologiques intracellulaires et des constituants des parois et membranes cellulaires obtenu par lyse de la souche de *Lactobacillus crispatus* selon l'invention.

Selon un mode particulier de l'invention, les fractions peuvent être le protoplasme de la bactérie.

Dans un mode de réalisation préférentiel de l'invention, les fractions seront associées au milieu de culture et / ou de fermentation de la bactérie.

Au sens de la présente invention, on entend par « métabolites » une ou plusieurs molécules organiques et / ou inorganiques issue du métabolisme de la souche de *Lactobacillus crispatus* selon la présente invention et dotée également d'une efficacité pour maintenir et / ou augmenter l'expression des molécules de la matrice extracellulaire de la peau saine et / ou des muqueuses saines, et / ou pour maintenir et / ou augmenter les propriétés biomécaniques de la peau saine et / ou des muqueuses saines. Préférentiellement, il s'agit du sécrétome de la souche de *Lactobacillus crispatus* selon l'invention.

Dans un mode de réalisation particulier de l'invention, les métabolites seront associés au milieu de culture et / ou de fermentation de la bactérie.

Au sens de la présente invention, on entend par « sécrétome », l'ensemble des molécules organiques sécrétées et / ou inorganiques sécrétées par la souche de *Lactobacillus crispatus* selon la présente invention doté d'une efficacité pour maintenir et / ou augmenter l'expression des molécules de la matrice extracellulaire de la peau saine et / ou des muqueuses saines, et / ou pour maintenir et / ou augmenter les propriétés biomécaniques de la peau saine et / ou des muqueuses saines.

Le sécrétome pourra être obtenu par toute méthode classiquement connue de l'homme du métier. Avantageusement, il sera obtenu selon le protocole tel que décrit par exemple dans l'exemple 1.d ou 1e.

Dans un mode de réalisation préférentiel de l'invention, le sécrétome sera associé au milieu de culture et / ou de fermentation de la bactérie.

Selon un mode préférentiel de réalisation de l'invention, la souche de *Lactobacillus crispatus* selon l'invention sera utilisée sous forme entière viable et / ou sous forme de lysat.

La souche de *Lactobacillus crispatus* selon l'invention pourra être issue de toute espèce connue de *Lactobacillus crispatus.* Préférentiellement, la souche de *Lactobacillus crispatus* est l'espèce déposée suivant le traité de Budapest auprès de l'Institut Pasteur (28 rue du Docteur Roux, F-75024 Paris cedex 15) le 09/09/2020 sous la désignation CNCM I-5579.

L'avantage de cette souche particulière de *Lactobacillus crispatus* est qu'elle est la première espèce de *Lactobacillus crispatus* retrouvée naturellement sur la peau saine.

Selon l'invention, la souche de *Lactobacillus crispatus* selon l'invention peut être utilisée seule, sous forme d'ingrédient actif et / ou dans une composition cosmétique et / ou nutraceutique, et / ou pharmaceutique, notamment dermatologique, préférentiellement destiné(e) à une application par voie topique et / ou par voie orale, encore préférentiellement à une application par voie topique.

Lorsqu'elle est utilisée sous forme entière vivante selon l'invention, et qu'elle est utilisée seule, sous forme d'ingrédient actif, la souche selon l'invention peut être sous forme sèche, c'est-à-dire sous forme de poudre, avantageusement dans de la maltodextrine, préférentiellement en une teneur en souche de 10% à 80% (p / p) en poids de la souche, préférentiellement de 30% à 70% (p / p), encore avantageusement de 40 à 60% (p/p) en poids de la souche par rapport au poids total de poudre.

Alternativement, la forme entière vivante selon l'invention peut être utilisée soluble et / ou diluée dans un solvant. Préférentiellement, ce solvant contient moins de 20% (v/v) en volume d'eau, encore préférentiellement moins de 5% (v/v) en volume d'eau, encore préférentiellement le solvant ne contient pas d'eau. Très préférentiellement, ce solvant est une huile cosmétiquement acceptable.

Lorsqu'elle est utilisée sous forme entière inactivée, notamment morte, et / ou sous forme de lysat, et / ou sous forme de l'une ou plusieurs de ses fractions, et / ou sous forme d'un ou plusieurs de ses métabolites, notamment de son sécrétome, et qu'elle est utilisée seule sous forme d'ingrédient actif, la souche selon l'invention peut être sous forme sèche, c'est-à-dire sous forme de poudre, avantageusement dans de la maltodextrine, préférentiellement en une teneur en souche de 5% à 80% (p / p) en poids de la souche, préférentiellement de 10% à 70%(p / p) en poids de la souche, très préférentiellement d'environ 20% à 50% (p / p) en poids de la souche par rapport au poids total de poudre, encore avantageusement de 10% à 50% (p/p) en poids de la souche par rapport au poids total de poudre.

Alternativement, les formes entières inactivées, notamment mortes, et / ou les lysats, et / ou les fractions, et / ou les métabolites, notamment le sécrétome selon l'invention, peuvent être utilisés solubles et / ou dilués dans un solvant, notamment polaire, tel que l'eau, un alcool, un polyol, un glycol tel que le pentylène glycol et / ou l'hexylène glycol et / ou le caprylyl glycol, ou un de leurs mélanges, préférentiellement un mélange hydroglycolique ou hydroalcoolique, encore préférentiellement comprenant un glycol choisi parmi l'hexylène glycol, le caprylyl glycol et leurs mélanges.

Dans un autre mode de réalisation, la souche selon l'invention peut être incorporée dans une composition cosmétique et / ou nutraceutique comprenant au moins un excipient cosmétiquement acceptable et / ou nutraceutiquement acceptable. Préférentiellement, elle est incorporée dans une composition cosmétique comprenant au moins un excipient cosmétiquement acceptable.

On entend, au sens de la présente invention, par excipient « cosmétiquement acceptable », un composé et / ou solvant topiquement acceptable, c'est-à-dire n'induisant pas de réponse allergique au contact de la peau, incluant le cuir chevelu humain, et / ou les muqueuses, non toxique, non instable, chimiquement.

Dans un mode de réalisation préférentiel de l'invention, la souche selon l'invention est présente dans la composition cosmétique et / ou dermatologique en une teneur comprise entre 1x10⁻⁴% et 10% (v / v) en volume, préférentiellement entre 1x10⁻⁴% et 5% (v / v) en volume, encore avantageusement entre 1x10⁻³% et 3% (v / v) en volume, encore préférentiellement entre 0,1% et 2% (v / v) en volume, par rapport au volume total de la composition, ladite composition comprenant en outre au moins un excipient cosmétiquement acceptable.

Avantageusement, la souche selon l'invention est présente dans la composition cosmétique et / ou dermatologique, à une concentration de 1x10⁻⁴% à 10% en poids, préférentiellement entre 1x10⁻⁴% et 5% en poids, encore avantageusement entre 1×10⁻³% et 0,5% en poids, par rapport au poids total de la composition, ladite composition comprenant en outre au moins un excipient cosmétiquement acceptable.

De manière particulièrement avantageuse, lorsque la souche selon l'invention est utilisée sous forme d'un ou plusieurs de ses métabolites, notamment de son sécrétome, elle est présente dans la composition cosmétique et / ou dermatologique, à une concentration de 0,05 à 0,5% en poids, encore préférentiellement à une concentration d'environ 0,1% en poids, par rapport au poids total de la composition, ladite composition comprenant en outre au moins un excipient cosmétiquement acceptable.

De manière particulièrement avantageuse, lorsque la souche selon l'invention est utilisée sous forme entière viable, elle est présente dans la composition cosmétique et / ou dermatologique, à une concentration de 0,01 à 0,1% en poids, encore préférentiellement à une concentration d'environ 0,025% en poids, par rapport au poids total de la composition, ladite composition comprenant en outre au moins un excipient cosmétiquement acceptable.

La composition cosmétique et / ou dermatologique de l'invention peut être choisie parmi une solution, aqueuse ou huileuse, une crème ou un gel aqueux ou un gel huileux, notamment un gel douche, un shampoing ; un lait ; une émulsion, une microémulsion ou une nanoémulsion, notamment huile-dans-eau ou eau-dans-huile ou multiple ou siliconée ; un masque ; un sérum ; une lotion ; un savon liquide ; un pain dermatologique ; une pommade ; un baume ; un beurre ; une mousse ; un patch ; un produit anhydre, de préférence liquide, pâteux ou solide, par exemple sous forme de poudres de maquillage, de bâtonnet ou de stick, notamment sous forme de rouge à lèvres.

Il peut également s'agir d'un produit de maquillage ou d'un produit de démaquillage.

De façon avantageuse, la souche de *Lactobacillus crispatus* ou la composition de l'invention est destinée à être appliquée sur tout ou partie du corps et / ou du visage et / ou du cuir chevelu, préférentiellement les jambes, les cuisses, les bras, le ventre, le décolleté, le cou, les aisselles, les lèvres, encore préférentiellement tout ou partie du visage, et préférentiellement les joues, le front, le menton, les lèvres, le contour des yeux.

De façon avantageuse, la souche et / ou la composition la comprenant est destinée à être appliquée sur une zone de peau saine présentant un relâchement et / ou une zone de peau saine affaissée et / ou une zone de peau saine manquant de tonicité.

Ainsi, avantageusement la composition cosmétique est destinée à une application par voie topique sur la peau saine et / ou les muqueuses saines.

De manière préférentielle, la souche de *Lactobacillus crispatus* selon l'invention est particulièrement adaptée pour la formulation de composition dite neutre et douce pour le respect de la glande sébacée, notamment de la peau incluant le cuir chevelu et / ou des muqueuses.

De manière alternative, la souche de *Lactobacillus crispatus* selon l'invention peut se présenter sous toutes les formes galéniques classiquement utilisées pour une application par voie orale, notamment sous forme d'ingrédient actif nutraceutique et / ou de composition nutraceutique.

Les compositions selon l'invention peuvent contenir tout solvant approprié et / ou tout véhicule approprié et / ou tout excipient approprié, éventuellement en combinaison avec d'autres composés d'intérêt.

De ce fait, pour ces compositions, l'excipient contient par exemple au moins un composé choisi parmi le groupe comprenant les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxydases, les stabilisants à base de sucrose, les vitamines E et ses dérivés, les cires naturelles et synthétiques, les huiles végétales, les triglycérides, les insaponifiables, les phytostérols, les esters végétaux, les silicones et ses dérivés, les hydrolysats de protéines, l'huile de Jojoba et ses dérivés, les esters lipo/hydrosolubles, les bétaïnes, les aminoxydes, les extraits de plantes, les esters de Saccharose, les dioxydes de Titane, les glycines, et les parabens, et encore de préférence parmi le groupe consistant en le butylène glycol, le stéareth-2, le stéareth-21, le glycol-15 stéaryl éther, le cétéaryl alcool, le phénoxyéthanol, le méthylparaben, l'éthylparaben, le propylparaben, le butylparaben, le butylène glycol, les tocophérols naturels, la glycérine, le dihydroxycétyl sodium phosphate, l'isopropyl hydroxycétyl éther, le glycol stéarate, la triisononanoïne, l'octyl cocoate, le polyacrylamide, l'isoparaffine, le laureth-7, un carbomer, le propylène glycol, le glycérol, le bisabolol, une diméthicone, l'hydroxyde de sodium, le PEG 30-dipolyhydroxystérate, les triglycérides caprique/caprylique, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, la cire d'abeille, les glycérides d'huile de cœur de palme hydrogénée, les glycérides d'huile de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée, et leurs mélanges.

De nombreux ingrédients cosmétiquement actifs sont connus par l'homme du métier pour améliorer la santé et / ou l'apparence physique de la peau (y compris le cuir chevelu) et / ou des muqueuses. L'homme du métier sait formuler les compositions cosmétiques, nutraceutiques ou dermatologiques pour obtenir les meilleurs effets. D'autre part, ces composés peuvent avoir un effet de synergie lorsqu'ils sont combinés les uns aux autres. Ces combinaisons sont également couvertes par la présente invention. Le CTFA Cosmetic Ingredient Handbook, Second Edition (1992) décrit différents ingrédients cosmétiques et pharmaceutiques utilisés couramment dans l'industrie cosmétique et pharmaceutique, qui sont en particulier adaptés à une utilisation topique. Des exemples de ces classes d'ingrédients comprennent, sans en être limités, les composés suivants: abrasifs, absorbants, composés à but esthétique tel que les parfums, les pigments, les colorants, les huiles essentielles, les astringents, (par exemple: l'huile de clou de girofle, menthol, camphre, l'huile d'eucalyptus, eugénol, menthyl lactate, distillat d'hamamélis), les agents anti-acné, les agents anti-floculants, les agents antimousse, les agents antimicrobiens (par exemple: iodopropyl butylcarbamate), les antioxydants, les liants, les additifs biologiques, les agents tampons, les agents gonflants, les agents chélatants, les additifs, les agents biocides, les dénaturants, les épaississants, et les vitamines, et les dérivés ou équivalents de ceux-ci, les matériaux formant des films, les polymères, les agents opacifiants, les ajusteurs de pH, les agents réducteurs, les agents dépigmentants ou éclaircissants (par exemple : hydroquinone, acide kojique, acide ascorbique, magnésium ascorbyl phosphate, ascorbyl glucosamine), les agents de conditionnement (par exemple : les humectants).

La composition cosmétique pourra comprendre par ailleurs d'autres agents cosmétiques et / ou nutraceutiques possédant les mêmes propriétés et induisant un effet synergique ou non avec la souche de *Lactobacillus crispatus* selon l'invention, ou des agents cosmétiques à effets complémentaires.

Il peut s'agir par exemple d'ingrédients anti-rides, agents stimulant l'activité ou la prolifération des fibroblastes, agents stimulant les molécules de la matrice extra-cellulaire.

Il peut s'agir par ailleurs d'ingrédients actifs anti-âge et / ou agents tenseurs pour un effet de synergie avec la souche de *Lactobacillus crispatus.* Avantageusement, il s'agit d'ingrédients actifs augmentant l'expression génique et / ou protéique du collagène ou d'ingrédients actifs empêchant la dégradation du collagène tels que le rétinol, la vitamine C, un extrait de *Davilla rugosa* commercialisé sous le nom de Collguard^{™} par BASF Beauty Care Solutions, un extrait de graine d*'Hibiscus abelmoschus* commercialisé sous le nom de Linefactor^{™}, un peptide commercialisé sous le nom de Dermican^{™} par la demanderesse ou encore les produits commercialisés sous les noms de Matrixyl^{™}, Matrixyl 3000^{™} et Regestril^{™} par la société Sederma ou Neuroguard par la société Codif, ou encore Eternaline^{™} par la société Silab.

Les agents tenseurs utilisables dans l'invention peuvent être choisis parmi les polymères synthétiques, tels que les latex de polyuréthanne ou les latex acryliques, les polymères d'origine naturelle, notamment les polyholosides sous forme d'amidon ou sous forme de carraghénanes, alginates, agars, gellanes, polymères cellulosiques et pectines; les protéines et hydrolysats de protéines végétales ; les silicates mixtes ; les microparticules de cire ; les particules colloïdales de charge inorganique choisies par exemple parmi la silice, les composites silice-alumine ; ainsi que leurs mélanges.

Il peut enfin s'agir d'ingrédients cosmétiques comme par exemple des agents antimicrobiens, agents anti-radicalaires, agents apaisants, calmants ou relaxants, agents agissant sur la microcirculation pour améliorer l'éclat du teint, en particulier du visage, agents cicatrisants ou agents amincissants. Avantageusement la composition cosmétique et / ou dermatologique de la présente invention contient également un ou plusieurs agents tenseurs et / ou un ou plusieurs agents antimicrobiens et / ou un ou plusieurs agents antiradicalaires et / ou un ou plusieurs agents apaisants et / ou un ou plusieurs agents amincissants et / ou un ou plusieurs agents actifs sur la microcirculation.

Parmi les agents antimicrobiens associés à la souche de *Lactobacillus crispatus* dans un mode préférentiel la présente invention, on peut citer le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, la terbinafine, l'acide undécylénique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octoxyglycérine, l'octanoylglycine, le caprylyl glycol, l'acide 10-hydroxy-2-décanoïque, le farnesol, les phytosphingosines et leurs mélanges.

Les agents anti-radicalaires peuvent être la vitamine C et ses dérivés dont le glucoside d'ascorbyle, les phénols et polyphénols, en particulier les tannins, l'acide ellagique et l'acide tannique; l'épigallocatéchine et les extraits naturels en contenant, en particulier les extraits de thé vert; les anthocyanes; les acides phénols, les stilbènes; des actifs piégeurs de composés aromatiques mono- ou polycycliques, les tannins tels que l'acide ellagique et les dérivés indoles et / ou des actifs piégeurs de métaux lourds tels que l'EDTA, des actifs anti-radicalaires tels que la vitamine E et ses dérivés tels que l'acétate de tocophéryle ; les bioflavonoïdes; la coenzyme Q10 ou ubiquinone.

Comme agents apaisants entrant dans la composition de l'invention, on peut utiliser les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de *Pueraria lobata* commercialisé sous le nom Inhipase^{™} par la demanderesse, les extraits de Theobroma cacao.

Les ingrédients actifs agissant sur la microcirculation, vasoprotecteurs ou vasodilatateurs, peuvent être choisis parmi les flavonoïdes, les ruscogénines, les nicotinates, les huiles essentielles.

Les actifs amincissants peuvent être notamment choisis parmi les agents inhibiteurs de la lipoprotéine lipase tels que ceux décrits dans le brevet US2003086949 (Coletica) et en particulier un extrait de liane du Pérou (*Uncaria tomentosa*); les actifs drainants, notamment l'hesperitine laurate (Flavagrum^{™}), ou quercitine caprylate (Flavenger^{™}), les agents inhibiteurs de l'enzyme phosphodiestarase, les agents activateurs de l'adenylate cyclase, l'AMPc et / ou les actifs capable de piéger la spermine et / ou la spermidine. On peut citer un extrait de racine de *Coleus Forskohlii,* un extrait de *Cecropia obtusa,* d*'Uva lactuca,* la caféine, la forskoline, la théophylline, la théobromine et / ou leurs dérivés, un produit de kappa carraghénanes hydrolysé dénommé Slimexcess^{™} commercialisé par la demanderesse et / ou leurs mélanges.

La présente invention a également pour objet un procédé de soin cosmétique comprenant l'application par voie topique sur au moins une zone de peau saine et / ou de muqueuse saine d'une souche de *Lactobacillus crispatus* selon l'invention, ou d'une composition cosmétique la comprenant, pour maintenir et / ou augmenter l'expression des molécules de la matrice extracellulaire de la peau saine et / ou des muqueuses saines, préférentiellement pour maintenir et / ou augmenter l'expression des collagènes de la peau saine et / ou des muqueuses saines et / ou des fibres élastiques de la peau saine et / ou des muqueuses saines, et / ou pour maintenir et / ou augmenter les propriétés biomécaniques de la peau saine et / ou des muqueuses saines, notamment la fermeté et / ou l'élasticité et / ou la densité, préférentiellement la fermeté.

De manière avantageuse, la souche de *Lactobacillus crispatus* selon l'invention, préférentiellement sous la forme d'une composition cosmétique selon l'invention, est utilisée en application topique régulière et préférentiellement au moins une fois par jour, avantageusement deux fois par jour, pendant au moins 10 jours, préférentiellement pendant 20 jours, et encore préférentiellement pendant au moins 28 jours.

De manière avantageuse, l'application par voie topique d'une souche de *Lactobacillus crispatus* selon l'invention ou d'une composition cosmétique la comprenant, est effectuée sur tout ou partie du corps et / ou du visage et / ou du cuir chevelu, préférentiellement les jambes, les cuisses, les bras, le ventre, le décolleté, le cou, les aisselles, les lèvres, encore préférentiellement tout ou partie du visage, et préférentiellement les joues, le front, le menton, les lèvres, le contour des yeux.

Le procédé de soin cosmétique selon l'invention pour maintenir et / ou augmenter l'expression des molécules de la matrice extracellulaire de la peau saine et / ou des muqueuses saines, préférentiellement pour maintenir et / ou augmenter l'expression des collagènes de la peau saine et / ou des muqueuses saines et / ou des fibres élastiques de la peau saine et / ou des muqueuses saines, et / ou pour maintenir et / ou augmenter les propriétés biomécaniques de la peau saine et / ou des muqueuses saines, notamment la fermeté et / ou l'élasticité et / ou la densité, préférentiellement la fermeté, comprend avantageusement les étapes suivantes :
- L'identification sur l'individu d'une zone de peau saine et / ou de muqueuse saine, pour laquelle on souhaite maintenir et / ou augmenter l'expression des molécules de la matrice extracellulaire de la peau saine et / ou des muqueuses saines, préférentiellement maintenir et / ou augmenter l'expression des collagènes de la peau saine et / ou des muqueuses saines et / ou des fibres élastiques de la peau saine et / ou des muqueuses saines, et / ou maintenir et / ou augmenter les propriétés biomécaniques de la peau saine et / ou des muqueuses saines, notamment la fermeté et / ou l'élasticité et / ou la densité, préférentiellement la fermeté, et
- L'application topique sur cette zone de peau saine et / ou de muqueuse saine, d'une composition cosmétique comprenant la souche de *Lactobacillus crispatus* selon l'invention, en une quantité efficace pour maintenir et / ou augmenter l'expression des molécules de la matrice extracellulaire de la peau saine et / ou des muqueuses saines, préférentiellement pour maintenir et / ou augmenter l'expression des collagènes de la peau saine et / ou des muqueuses saines et / ou des fibres élastiques de la peau saine et / ou des muqueuses saines, et / ou pour maintenir et / ou augmenter les propriétés biomécaniques de la peau saine et / ou des muqueuses saines, notamment la fermeté et / ou l'élasticité et / ou la densité, préférentiellement la fermeté, à savoir en une teneur de souche de *Lactobacillus crispatus* comprise :
- entre 1x10⁻⁴% et 10% (v / v) en volume, préférentiellement entre 1x10⁻⁴% et 5% (v / v) en volume, encore avantageusement entre 1x10⁻³% et 3% (v / v) en volume, encore préférentiellement entre 0,1% et 2% (v / v) en volume, par rapport au volume total de la composition, et/ou
- entre 1x10⁻⁴% et 10% (p / p) en poids, préférentiellement entre 1x10⁻⁴% et 5% (p / p) en poids, encore avantageusement entre 1x10⁻³% et 0,5% (p / p) en poids, par rapport au poids total de la composition.

Au sens de la présente invention, on entend par « voie topique », l'application locale directe et / ou la vaporisation de la souche de *Lactobacillus crispatus* ou de la composition selon l'invention sur la surface de la zone de peau saine et / ou de muqueuse saine.

Au sens de la présente invention, on entend par « topiquement et / ou oralement acceptable », un ingrédient adapté à une application respectivement par voie topique et / ou orale, non toxique, non irritant pour la peau y compris le cuir chevelu et n'induisant pas de réponse allergique, et qui n'est pas instable sur le plan chimique.

L'invention a également pour objet la souche de *Lactobacillus crispatus* selon l'invention, seule ou dans une composition dermatologique la comprenant, pour son utilisation, avantageusement par voie topique pour le traitement et / ou la prévention des pathologies de la peau et / ou des muqueuses impliquant une diminution de la quantité et/ou l'expression des molécules de la matrice extracellulaire de la peau et / ou des muqueuses, et / ou une diminution de la quantité et/ou de l'expression des collagènes et / ou des fibres élastiques de la peau et / ou des muqueuses, et / ou une diminution de la quantité et/ou des propriétés biomécaniques de la peau et / ou des muqueuses, notamment une diminution de la fermeté de la peau et / ou des muqueuses et/ou une diminution d'élasticité de la peau et / ou des muqueuses, et/ou une diminution de la densité de la peau et / ou des muqueuses.

Au sens de la présente invention, on entend d'un point de vue dermatologique par « traitement et / ou prévention des pathologies de la peau et / ou des muqueuses impliquant une diminution de la fermeté de la peau et / ou des muqueuses », une augmentation à des fins thérapeutiques de la fermeté de la peau et / ou des muqueuses qui ont perdu de la fermeté sous l'effet de pathologies de la peau et / ou des muqueuses comme par exemple la couperose, les télangiectasies.

Au sens de la présente invention, on entend d'un point de vue dermatologique par « traitement et / ou prévention des pathologies de la peau et / ou des muqueuses impliquant une diminution d'élasticité de la peau et / ou des muqueuses », une augmentation à des fins thérapeutiques de l'élasticité de la peau et / ou des muqueuses qui ont perdu de leur élasticité sous l'effet de pathologies de la peau et / ou des muqueuses comme par exemple l'élastose solaire et / ou la pathologie cutis laxa.

Au sens de la présente invention, on entend d'un point de vue dermatologique par « traitement et / ou prévention des pathologies de la peau et / ou des muqueuses impliquant une diminution de densité de la peau et / ou des muqueuses » une augmentation à des fins thérapeutiques de la densité de la peau et / ou des muqueuses qui ont perdu de leur densité sous l'effet de pathologies de la peau et / ou des muqueuses comme par exemple le syndrome Ehlers-Danlos et / ou la dermatoporose. La souche de *Lactobacillus crispatus* selon l'invention peut se trouver sous la forme d'une composition pharmaceutique, préférentiellement dermatologique, comprenant au moins un excipient pharmaceutiquement et / ou dermatologiquement, acceptable. Dans un mode de réalisation de l'invention, ladite composition est appliquée par voie topique et / ou orale, préférentiellement par voie topique.

Avantageusement, elle est présente dans la composition pharmaceutique, préférentiellement dermatologique, à une concentration de 1x10⁻⁴% à 10% en poids, préférentiellement entre 1x10⁻⁴% et 5% en poids, encore avantageusement entre 1×10⁻³% et 0,5% en poids par rapport au poids total de la composition, ladite composition comprenant en outre au moins un excipient pharmaceutiquement acceptable.

Avantageusement, la souche de *Lactobacillus crispatus* selon l'invention est présente dans la composition pharmaceutique, préférentiellement dermatologique, à une concentration de 1x10⁻⁴% à 10% (v / v) en volume, préférentiellement de 1x10⁻⁴% à 5% (v / v) en volume, encore avantageusement de 1x10⁻³% à 0,5% (v / v) en volume, encore préférentiellement entre 0,1% et 2% (v / v) en volume, par rapport au volume total de la composition, ladite composition comprenant en outre au moins un excipient pharmaceutiquement acceptable.

L'invention sera mieux comprise à la lecture de la description et des exemples qui suivent.

Des exemples faisant référence à la description de l'invention sont présentés ci-après. Ces exemples sont donnés à titre d'illustration et ne sauraient limiter en aucun cas la portée de l'invention. Chacun des exemples a une portée générale.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention.

D'autre part, dans les exemples, tous les pourcentages sont donnés en poids, sauf indication contraire, la température est exprimée en degré Celsius sauf indication contraire, et la pression est la pression atmosphérique, sauf indication contraire.

### Exemple 1. Préparation de différentes formes de Lactobacillus crispatus

### Exemple 1.a : production de forme entière viable de L. crispatus.

Les bactéries *L. crispatus* (CNCM I-5579) isolées ont été ensemencées dans un milieu de culture de type MRS, incubées à 37°C en anaérobie. Le pH a été maintenu à une valeur de pH = 6, et le milieu a été incubé 24h. En fin d'incubation, le milieu a été centrifugé, le concentré cellulaire a été resuspendu dans le milieu de fermentation et lyophilisé en présence de maltodextrine, pour une quantité finale de maltodextrine de 70% (p/p) en poids par rapport au poids total de l'ingrédient.

### Exemple 1.b : production de la forme entière inactivée de L. crispatus

Les bactéries *L. crispatus* (CNCM I-5579) isolées ont été ensemencées dans un milieu de culture de type MRS, incubées à 37°C en anaérobie. Le pH a été maintenu à une valeur de pH = 6, et le milieu a été incubé 24h. En fin d'incubation le milieu est centrifugé, le concentré cellulaire est inactivé en le chauffant à 80°C pendant 30 minutes. Le concentré cellulaire a ensuite été re suspendu dans de la glycérine.

### Exemple 1.c : production du lysat de la bactérie L. crispatus

Les bactéries *L. crispatus* (CNCM I-5579) isolées ont été ensemencées dans un milieu de culture de type MRS, incubées à 37°C en anaérobie. Le pH a été maintenu à une valeur de pH = 6, et le milieu a été incubé 24h. En fin d'incubation l'ensemble est passé à l'homogénéisateur haute pression. Le lysat a ensuite été re suspendu dans de la glycérine.

### Exemple 1.d : production du sécrétome de la bactérie L. crispatus

Les bactéries *L. crispatus* (CNCM I-5579) isolées ont été ensemencées dans un milieu de culture de type MRS, incubées à 37°C en anaérobie. Le pH a été maintenu à une valeur de pH = 6, et le milieu a été incubé 24h. En fin d'incubation le milieu est filtré pour récupérer le sécrétome de la bactérie. Le sécrétome a ensuite été re suspendu dans de la glycérine.

### Exemple 1.e : production du sécrétome de la bactérie L. crispatus sous forme de poudre

Les bactéries *L. crispatus* (CNCM I-5579) isolées ont été ensemencées dans un milieu de culture de type MRS, incubées à 37°C en anaérobie. Le pH a été maintenu à une valeur de pH = 6, et le milieu a été incubé 24h. En fin d'incubation le milieu a été filtré pour récupérer le sécrétome de la bactérie. Le sécrétome a ensuite été atomisé avec de la maltodextrine, pour une quantité finale de maltodextrine de 90% (p/p) en poids par rapport au poids total de l'ingrédient.

### Exemple 1.f : production de forme entière viable de L. crispatus.

Les bactéries *L. crispatus* (CNCM I-5579) isolées ont été ensemencées dans un milieu de culture de type MRS, incubées à 37°C en anaérobie. Le pH a été maintenu à une valeur de pH = 6, et le milieu a été incubé 24h. En fin d'incubation, le milieu a été centrifugé, le concentré cellulaire a été resuspendu dans le milieu de fermentation et lyophilisé en présence de maltodextrine, pour une quantité finale de maltodextrine de 50% (p/p) en poids par rapport au poids total de l'ingrédient.

### Exemple 2 : Augmentation de l'expression de collagène de type I par les fibroblastes en présence la souche de L. crispatus selon l'invention.

Des fibroblastes humains dits « normaux », c'est-à-dire ne présentant pas de pathologie, issus d'une donneuse saine de 34 ans ont été ensemencés en plaque 24 puits puis cultivés dans un milieu DMEM jusqu'à confluence.

L'incubation a été poursuivie 48 heures en présence des bactéries *L. crispatus* selon l'exemple 1.a ensemencées dans des nacelles au-dessus des puits de culture dans un milieu MRS dilué avec du PBS à une concentration de 10⁶ CFU/mL. Le même milieu de culture sans ajout de la souche selon l'invention a été utilisé comme témoin (Contrôle).

En fin d'incubation, les bactéries ont été éliminées et les cellules ont été lysées à l'aide d'un tampon hydroxyde d'ammonium. Le tampon a ensuite été éliminé, et les puits ont été saturés avec un mélange de PBS/ BSA (Phosphate Buffer Saline / Bovine sérum albumine).

Après saturation, l'anticorps primaire anti-collagène I (Interchim / Acris, Montluçon, France, LH0284 / R1038) a été incubé quelques minutes, suivi de l'anticorps secondaire conjugué Europium (Perkin Elmer) la fluorescence est ensuite mesurée à (λexc. 340 nm / λem. 615 nm) en utilisant un lecteur de plaque à étiquettes multiples EnVision^{®} (Perkin Elmer).

Les résultats de fluorescence ont été normalisés par rapport à la fluorescence obtenue avec le même milieu cellulaire en l'absence la souche de *L. crispatus* selon l'invention (Contrôle) et ont été rapportés à la quantité d'ADN obtenue dans chaque condition. Les résultats présentés correspondent à la moyenne de 6 essais (n=6).

### Résultats

| | Contrôle (milieu de culture) | Souche de L. crispatus selon l'exemple 1.a |
|---|---|---|
| Moyenne de stimulation (%) | 100 | 165 |
| Ecart type | 26 | 32 |
| Statistique /Control (One way ANOVA) | - | (*) p<0.05 |

La souche de *L.crispatus* selon l'exemple 1.a produit de l'invention a montré une stimulation significative du collagène I par les fibroblastes cutanés humain cultivés *in vitro.* Elle peut donc être utilisée pour augmenter la fermeté de la peau saine et / ou des muqueuses saines.

### Exemple 3 : Augmentation de l'expression des protéines Fibuline-5 et Emilin-1 impliquées dans la formation des fibres élastiques par les fibroblastes en présence la souche de L. crispatus selon l'invention.

Des fibroblastes humains dits « normaux » c'est-à-dire ne présentant pas de pathologie, issus d'une donneuse saine de 19 ans ont été cultivés dans un milieu FGM (Fibroblast Growth Medium). Une fois mis en culture, l'incubation a été poursuivie pendant 48h en présence du sécrétome de la bactérie *L.crispatus* obtenus selon l'exemple 1.e à une concentration de 0,1% (p/p) par rapport au poids total du milieu de culture et du sécrétome. Le même milieu de culture sans ajout de la souche selon l'invention a été utilisé comme témoin (Contrôle).

A la fin de l'incubation, le milieu de culture a été retiré et les cellules ont été lysées à l'aide d'un tampon de lyse.

La fibuline-5 et l'Emilin-1 ont été identifiées et quantifiées par un système d'analyse des protéines basé sur l'électrophorèse capillaire. Une quantité de protéines issue des lysats cellulaires, à savoir 0,2 mg/mL de lysat pour fibuline-5 et 0,2 mg/mL de surnageant du lysat pour Emilin-1, a été déposé dans des capillaires dans lesquels il a été ajouté un anticorps primaire anti-fibulin-5 ou un anticorps anti-Emilin-1 suivi d'un anticorps secondaire conjugué à la peroxydase de raifort et d'un substrat chemiluminescent. L'ensemble a été incubé 30 minutes, puis le signal chemiluminescent résultant de la détection de la protéine fibuline-5 a été quantifiée par électrophorèse capillaire à l'aide d'un logiciel adapté.

Les résultats sont la moyenne des essais effectués ± l'écart-type, exprimé en pourcentage, par rapport au contrôle non traité normalisé à 100%. L'analyse statistique des résultats a été faite par rapport au témoin non traité en utilisant le test One-Way ANOVA. Le seuil de significativité est placé à 5%.

### Résultats

| | Contrôle (Milieu de culture) | Sécrétome de L.crispatus selon l'exemple 1.e à 0.1% (p/p) |
|---|---|---|
| Moyenne de stimulation de la Fibuline-5 en % | 100 | 150 |
| Ecart -type | 8.2 | 16.6 |
| Statistiques | NA | P<0.001 |

Le sécrétome de la souche de *Lactobacillus crispatus* selon l'exemple 1.e de l'invention a montré une augmentation significative de 50% de l'expression protéique de la Fibuline-5 par les fibroblastes cutanés humains cultivés *in vitro.* Ainsi, la souche de *Lactobacillus crispatus,* et en particulier son sécrétome peut donc être utilisée pour augmenter l'élasticité de la peau saine et / ou des muqueuses saines.

| | Contrôle (Milieu de culture) | Sécrétome de L. crispatus selon l'exemple 1.e à 0.1% (p/p) |
|---|---|---|
| Moyenne de stimulation de Emilin-1 en % | 100 | 153.1 |
| Ecart -type | 12.7 | 15.1 |
| Statistiques | NA | P<0.001 |

Le sécrétome de la souche de *Lactobacillus crispatus* selon l'exemple 1.e de l'invention a montré une augmentation significative de 53% de l'expression protéique de l'Emilin-1 par les fibroblastes cutanés humains cultivés *in vitro.* Ainsi, la souche de *Lactobacillus crispatus* et en particulier son sécrétome peut donc être utilisée pour augmenter l'élasticité de la peau saine et / ou des muqueuses saines.

### Exemple 4 : Mesure in vivo de l'augmentation de l'élasticité de la peau en présence de la souche de L. crispatus selon l'invention.

Une population de 30 femmes âgées de 40 à 50 ans ayant une diminution de l'élasticité de la peau au niveau du visage a appliqué en hémi-visage une crème sous forme d'émulsion telle que décrite en exemple 7b), comprenant une concentration finale en poids du sécrétome de la souche *L. crispatus* selon l'exemple 1.e de 1% (p/p) par rapport au poids total de la crème ou sous forme de la même émulsion dite placebo dans laquelle ledit sécrétome a été remplacé par de l'eau, à raison de 2 applications par jour et pendant 2 mois.

L'évaluation des propriétés biomécaniques de la peau a été réalisée en utilisant un cutomètre^{®}. Cet équipement d'évaluation clinique à la capacité de mesurer 3 paramètres distincts que sont l'élasticité globale, l'élasticité immédiate, ainsi que la récupération élastique de la peau, qui vont tous 3 refléter l'élasticité de la peau. De ces mesures ont été déduits différents paramètres reflétant l'état de l'élasticité de la peau.

L'élasticité globale, immédiate, ainsi que la récupération élastique de la peau ont été mesurées par le cutomètre^{®} au début de l'étude (D0), après un mois d'application (D28) et enfin après 2 mois d'application (D56).

L'efficacité du sécrétome de la souche de *Lactobacillus crispatus* selon l'exemple 1.e a été comparée à celle obtenue avec l'émulsion placébo.

### Résultats

| | **Formulation placebo** | | **Formulation contenant la souche de L.crispatus selon l'exemple 1.e à 0.1% (p/p)** | |
|---|---|---|---|---|
| | A D28 | A D56 | A D28 | A D56 |
| Pourcentage d'amélioration de l'élasticité globale | 1.1% | 2.7% | +4.4% (p<0.001 vs D0) et p<0.05 vs placebo | +12% p<0.001 vs D0 et vs placebo) |
| Pourcentage d'amélioration de l'élasticité nette | 5.9% (p<0.05 vs D0) | 1.6% | 5.8% (p<0.05 vs D0) | 11.7% (p<0.001 vs D0 et vs Placebo) |
| Pourcentage d'amélioration de la récupération élastique | 2.9% | 2.7% | 4.7% (p<0.001 vs D0) | 12.5% (p<0.001 vs D0 et vs Placebo) |

Après 2 mois d'utilisation, la formulation contenant le sécrétome de la souche de *Lactobacillus crispatus* selon l'exemple 1.e a induit une augmentation significative de tous les paramètres de l'élasticité. Cette augmentation est significative par rapport au début de l'étude DO mais également par rapport au placebo.

Ces résultats montrent l'effet bénéfique de la souche de *Lactobacillus crispatus* mise en formulation, en particulier son sécrétome pour maintenir et / ou augmenter in vivo les propriétés biomécaniques de la peau saine et / ou des muqueuses saines, en particulier l'élasticité de la peau saine et / ou des muqueuses saines.

### Exemple 5 : Mesure in vivo de l'augmentation de la densité de la peau en présence de la souche de L. crispatus selon l'invention.

Une population de 31 femmes âgées de 45 à 65 ans ayant des rides au niveau du front et une diminution des propriétés biomécanique de la peau au niveau du visage a appliqué en hémi-visage une crème sous forme d'émulsion telle que décrite dans la formulation présentée en exemple 7.c, comprenant une concentration finale en poids de la souche *L. crispatus* obtenue selon l'exemple 1.f de 0,05% (p/p) par rapport au poids total de la crème ou sous forme de la même émulsion dite placebo dans laquelle ladite souche a été remplacée par de l'eau, à raison de 2 applications par jour et pendant 2 mois.

L'évaluation de la densité de la peau a été réalisée à l'aide d'un Scanner équipé d'une sonde ultrason. Le Scanner permet de visualiser la densité de la peau qui est le reflet de la synthèse des composés de la matrice extracellulaire.

L'amélioration de la densité de la peau ainsi que de son épaisseur ont été mesurées au début de l'étude (D0) après un mois d'application (D28) et enfin après 2 mois d'application (D56).

L'efficacité de la souche de *Lactobacillus crispatus* selon l'exemple 1.f a été comparée à celle d'une émulsion placébo.

### Résultats

| | **Formulation placebo** | **Formulation contenant la souche de L.crispatus selon l'exemple 1.f à 0.05% (p/p)** |
|---|---|---|
| | A D56 | A D56 |
| Pourcentage d'augmentation de la densité du derme vs D0 | Non significatif | +6% (p<0.05 vs D0 et Vs placebo) |

Après 2 mois d'utilisation, la formulation de la souche de *Lactobacillus crispatus* selon l'exemple 1.f a induit une augmentation significative de la densité de la peau. Cette augmentation est significative par rapport au début de l'étude DO mais également par rapport au placebo.

Ces résultats montrent l'effet bénéfique de la souche de *Lactobacillus crispatus* mise en formulation pour maintenir et / ou augmenter in vivo les propriétés biomécaniques de la peau saine et / ou des muqueuses saines, en particulier la densité de la peau saine et / ou des muqueuses saines.

### Exemple 6 : Exemple d'ingrédient cosmétique comprenant la souche de Lactobacillus crispatus selon l'invention

| | |
|---|---|
| Extrait *Lactobacillus crispatus* selon l'exemple 1.c | 0,2% |
| Glycérine | 80% |
| Eau | QSP 100 |

### Exemple 7 : Exemple de composition cosmétique comprenant la souche de Lactobacillus crispatus selon l'invention

**Exemple 7.a :** Exemple de composition cosmétique comprenant la souche de *Lactobacillus crispatus* selon l'exemple 1.c

On procède selon les méthodes connues de l'homme du métier pour mélanger ensemble les différentes phases A, B, C, D ci-dessous pour préparer une composition selon la présente invention. Les proportions sont exprimées en % et les noms en majuscules correspondent aux noms INCI des ingrédients.

### Phase A

| | |
|---|---|
| GLYCERYL STEARATE AND PEG-100 STEARATE | 4,00 |
| PENTAERYTHRITYL DISTEARATE | 1,50 |
| CETEARYL ISONONANOATE | 3,00 |
| PROPYLHEPTYL CAPRYLATE | 5,00 |
| COCO CAPRYLATE | 2,00 |
| DICAPRYLYL CARBONATE | 3,00 |
| DIMÉTHICONE | 1,00 |

### Phase B

| | |
|---|---|
| Eau | 64,43 |
| Propylène glycol, phénoxyéthanol, chlorphénésine, méthylparabène | 1,00 |
| Glycérine | 1,57 |
| Gomme de xanthane | 0,20 |
| Butylène glycol | 2,00 |
| Hydroxyde de sodium, eau | 0,15 |

### Phase C

| | |
|---|---|
| Carbomère | 0,15 |
| Eau | 10 |

### Phase D

| | |
|---|---|
| Extrait de *Lactobacillus crispatus* obtenu selon exemple 1c) | 1,00 |

**Exemple 7.b :** Exemple de composition cosmétique comprenant la souche de *Lactobacillus crispatus* selon l'exemple 1.e

Les proportions sont exprimées en % et les noms en majuscules correspondent aux noms INCI des ingrédients.

### Phase A

| | |
|---|---|
| GLYCERIN | 5,00 |
| BUTYLENE GLYCOL | 10,00 |
| XANTHAN GUM | 2,00 |

### Phase B

| | |
|---|---|
| Eau | 58.95 |
| Sodium benzoate | 0,25 |
| GLYCERINE, EAU, SODIUM LEVULINATE, SODIUM ANISATE | 1,00 |

### Phase C

| | |
|---|---|
| POLYGLYCERYL-2 DIPOLYHYDROXYSTEARATE | 1,00 |
| DIPROPYLHEPTYL CARBONATE | 3,00 |
| DICAPRYLYL CARBONATE | 3,00 |
| CAPRYLYL CAPRYLATE/CAPRATE | 10,00 |
| LAURETH-7 CITRATE | 0,50 |

### Phase D

| | |
|---|---|
| Acide citrique | 0,30 |

### Phase E

| | |
|---|---|
| Extrait de *Lactobacillus crispatus* obtenu selon exemple 1e | 1,00 |
| Eau | 4,00 |

On procède selon les méthodes connues de l'homme du métier pour mélanger ensemble les différentes phases A, B, C à température ambiante, en ajoutant B en A en remuant, puis C dans AB. On ajuste le pH à 4,9 avec D. Puis on ajoute la phase E. Enfin, on homogénéise par Ultra Turrax pour préparer une composition selon la présente invention.

**Exemple 7.c :** Exemple de composition cosmétique comprenant la souche de *Lactobacillus crispatus* selon l'exemple 1.f

La souche de *Lactobacillus crispatus obtenue* selon l'exemple 1.f est ajoutée juste avant l'application sur la peau dans une formulation telle que défini ci-dessous selon la Table 8, à une concentration finale de 0,05% (p/p) par rapport au poids total de la formulation.

| Dénomination | Quantité (% en poids total) |
|---|---|
| **Phase A** | |
| Ceterayl Alcohol, Lecithin, Sodium Cetearyl Sulfate, olus oil | 3.00 |
| Hydrogenated Vegetable Glycerides | 2.50 |
| Caprylyl Caprylate/Caprate | 6.50 |
| Dicaprylyl Carbonate | 4.00 |

| **Phase B** | |
|---|---|
| Water | 66.35 |
| Butylene Glycol | 10.00 |
| Sodium Benzoate | 0.25 |

| **Phase C** | |
|---|---|
| Glycerin | 5.00 |
| Xanthan Gum | 1.00 |

| | |
|---|---|
| | |

| **Phase D** | |
|---|---|
| Glycerin, Aqua, Sodium Levulinate, Sodium Anisate | 1.00 |
| Citric Acid | 0.40 |

### Exemple 8 : Exemple d'une composition dermatologique

### Exemple 8a) : Pommade contenant la souche entière viable de L. crispatus selon l'invention.

La composition ci-après est préparée selon des méthodes connues de l'homme du métier, en particulier s'agissant des différentes phases à mélanger entre elles.

Les quantités indiquées sont en pourcentage en poids par rapport au poids total de la composition.

| | |
|---|---|
| Ingrédient * | 3,00 |
| Excipient : | |
| Polyéthylène basse densité | 5,50 |
| Paraffine liquide | Qsp 100 |

| | |
|---|---|
| * L'ingrédient est préparé selon l'exemple 1a) précédent. L'ingrédient selon l'invention est stérilisé puis séché avant d'être incorporé dans la composition sous forme de pommade. | |

### Exemple 8b) : Crème contenant la souche de Lactobacillus crispatus selon l'exemple 1c).

| Phase | Dénomination | Quantité (% en poids total) |
|---|---|---|
| A | Eau | 79,60 |
| A | PROPYLENE GLYCOL (AND) PHENOXYETHANOL (AND) CHLORPHENESIN (AND) METHYLPARABEN | 2,50 |
| A | Glycérine | 2 |
| A | SODIUM STEAROYL GLUTAMATE | 0,5 |
| B | DICAPRYLYL CARBONATE | 3 |
| B | COCO-CAPRYLATE / CAPRATE | 3 |
| B | CAPRYLIC / CAPRIC TRIGLYCERIDE | 3 |
| B | SUCROSE POLYSTEARATE (AND) CETYL PALMITATE | 3 |
| B | PENTAERYTHRITYL DISTEARATE | 1 |
| B | SODIUM POLYACRYLATE | 0,7 |
| C | Eau | 0,9 |
| C | Extrait de *Lactobacillus crispatus* selon l'exemple 1c) | 0,1 |
| D | Acide citrique et eau | 0,7 |

Les phases A et B sont chauffées à 75° puis mélangés sous agitation. Une fois que le mélange est revenu à température ambiante, les phases C et D sont ajoutées sous agitation.

## Revendications

1. Utilisation cosmétique et / ou nutraceutique d'une souche de *Lactobacillus crispatus,* préférentiellement la souche de *Lactobacillus crispatus* étant l'espèce déposée sous la désignation CNCM I-5579, pour maintenir et / ou augmenter l'expression des molécules de la matrice extracellulaire de la peau saine et / ou des muqueuses saines.

2. Utilisation selon la revendication 1, dans laquelle les molécules de la matrice extracellulaire de la peau saine et / ou des muqueuses saines sont choisies parmi les fibres de collagène de la peau saine et / ou des muqueuses saines, et / ou les fibres élastiques de la peau saine et / ou des muqueuses saines.

3. Utilisation selon la revendication 1 ou 2, pour maintenir et / ou augmenter l'expression des collagènes de type I, III, IV, V, VI, VII, XII, XIII, XIV, XVI, XVII, XXIV et / ou XXIX, préférentiellement de type I, III, IV, V et / ou XIV, préférentiellement les fibres de collagène de type I, IV et / ou V, encore préférentiellement les fibres de collagène de type I ; et/ou
pour maintenir et / ou augmenter les propriétés biomécaniques de la peau saine et / ou des muqueuses saines, préférentiellement la fermeté et / ou l'élasticité et / ou la densité de la peau saine et / ou des muqueuses saines, encore préférentiellement la fermeté et / ou l'élasticité de la peau saine et / ou des muqueuses saines.

4. Utilisation selon l'une quelconque des revendications précédentes, dans laquelle la souche est utilisée sous forme entière, notamment viable et / ou inactivée, notamment morte, et / ou sous forme de lysat, et / ou sous forme de l'une ou plusieurs de ses fractions, et / ou sous forme d'un ou plusieurs de ses métabolites, préférentiellement de son sécrétome.

5. Utilisation selon l'une quelconque des revendications précédentes, dans laquelle la souche est utilisée sous forme entière viable et / ou sous forme de lysat et / ou dans laquelle la souche utilisée est la souche de *Lactobacillus crispatus* déposée sous la désignation CNCM I-5579.

6. Utilisation selon l'une des revendications précédentes, dans laquelle la souche de *Lactobacillus crispatus* est associée à son milieu de fermentation et / ou de culture.

7. Utilisation selon l'une des revendications précédentes, dans laquelle la souche de *Lactobacillus crispatus* est appliquée par voie topique sur la peau saine et / ou les muqueuses saines.

8. Utilisation selon l'une quelconque des revendications précédentes, telle que la souche de *Lactobacillus crispatus* est présente dans la composition cosmétique à une concentration de 1x10-4% à 10% en poids, préférentiellement entre 1x10-4% et 5% en poids, encore avantageusement entre 1x10-3% et 0,5% en poids par rapport au poids total de la composition, ladite composition comprenant en outre au moins un excipient cosmétiquement acceptable.

9. Procédé de soin cosmétique **caractérisé en ce qu'**il comprend l'application par voie topique sur au moins une zone de peau saine et / ou de muqueuse saine d'une souche de *Lactobacillus crispatus* selon l'une des revendications 4, 5 ou 6, ou d'une composition cosmétique selon la revendication 8, pour maintenir et / ou augmenter l'expression des molécules de la matrice extracellulaire de la peau saine et / ou des muqueuses saines, préférentiellement pour maintenir et / ou augmenter l'expression des collagènes de la peau saine et / ou des muqueuses saines et / ou des fibres élastiques de la peau saine et / ou des muqueuses saines, et / ou pour maintenir et / ou augmenter les propriétés biomécaniques de la peau saine et / ou des muqueuses saines, notamment la fermeté et / ou l'élasticité et / ou la densité, préférentiellement la fermeté.

10. Procédé de soin cosmétique selon la revendication 9, dans laquelle l'application par voie topique d'une souche de *Lactobacillus crispatus* selon l'une des revendications 4, 5 ou 6, ou d'une composition cosmétique selon la revendication 8, est effectuée sur tout ou partie du corps et / ou du visage et / ou du cuir chevelu, préférentiellement les jambes, les cuisses, les bras, le ventre, le décolleté, le cou, les aisselles, les lèvres, encore préférentiellement tout ou partie du visage, et préférentiellement les joues, le front, le menton, les lèvres, le contour des yeux.

11. Procédé de soin cosmétique selon l'une quelconque des revendications 9 à 10, dans lequel la composition cosmétique comprend la souche de *Lactobacillus crispatus* selon l'une des revendications 4, 5 ou 6 à une concentration de 1x10-4% à 10% en poids, préférentiellement entre 1x10-4% et 5% en poids, encore avantageusement entre 1x10-3% et 0,5% en poids par rapport au poids total de la composition, ladite composition comprenant en outre au moins un excipient cosmétiquement acceptable.

12. Souche de *Lactobacillus crispatus,* ou une composition dermatologique la comprenant, pour son utilisation, avantageusement par voie topique, pour le traitement et / ou la prévention des pathologies de la peau et / ou des muqueuses impliquant une diminution de la quantité et/ou de l'expression des molécules de la matrice extracellulaire de la peau et / ou des muqueuses, et / ou une diminution de la quantité et/ou de l'expression des collagènes et / ou des fibres élastiques de la peau et / ou des muqueuses, et / ou une diminution des propriétés biomécaniques de la peau et / ou des muqueuses, notamment une diminution de la fermeté de la peau et / ou des muqueuses et / ou une diminution d'élasticité de la peau et / ou des muqueuses et/ou une diminution de la densité de la peau et/ou des muqueuses,
la souche étant utilisée sous forme entière, notamment viable et / ou inactivée, notamment morte, et / ou sous forme de lysat, et / ou sous forme de l'une ou plusieurs de ses fractions, et / ou sous forme d'un ou plusieurs de ses métabolites, préférentiellement de son sécrétome,
et/ou la souche étant associée à son milieu de fermentation et / ou de culture.

13. Souche de *Lactobacillus crispatus* pour son utilisation selon la revendication 12, **caractérisée en ce qu'**elle se trouve sous la forme d'une composition dermatologique comprenant au moins un excipient dermatologiquement acceptable.

14. Souche de *Lactobacillus crispatus* pour son utilisation selon l'une quelconque des revendications 12 à 13, **caractérisée en ce qu'**elle est présente dans la composition pharmaceutique, préférentiellement dermatologique, à une concentration de 1x10-4% à 10% en poids, préférentiellement entre 1x10-4% et 5% en poids, encore avantageusement entre 1x10-3% et 0,5% en poids par rapport au poids total de la composition, ladite composition comprenant en outre au moins un excipient pharmaceutiquement acceptable.

15. Souche de *Lactobacillus crispatus* pour son utilisation selon l'une quelconque des revendications 12 à 14, **caractérisée en ce que** la souche est selon l'une des revendications 4, 5 ou 6.

## Patentansprüche

1. Kosmetische und/oder nutrazeutische Verwendung eines *Lactobacillus crispatus-Stamms,* wobei vorzugsweise der *Lactobacillus crispatus-Stamm* die unter der Bezeichnung CNCM I-5579 hinterlegte Spezies ist, zum Aufrechterhalten und/oder Erhöhen der Expression von Molekülen der extrazellulären Matrix gesunder Haut und/oder gesunder Schleimhäute.

2. Verwendung nach Anspruch 1, wobei die Moleküle der extrazellulären Matrix gesunder Haut und/oder gesunder Schleimhäute aus Kollagenfasern gesunder Haut und/oder gesunder Schleimhäute und/oder elastischen Fasern gesunder Haut und/oder gesunder Schleimhäute ausgewählt sind.

3. Verwendung nach Anspruch 1 oder 2 zum Aufrechterhalten und/oder Erhöhen der Expression der Kollagene Typ I, III, IV, V, VI, VII, XII, XIII, XIV, XVI, XVII, XXIV und/oder XXIX, vorzugsweise Typ I, III, IV, V und/oder XIV, vorzugsweise der Fasern von Kollagen Typ I, IV und/oder V, noch stärker bevorzugt der Fasern von Kollagen Typ I, und/oder
zum Aufrechterhalten und/oder Erhöhen der biomechanischen Eigenschaften gesunder Haut und/oder gesunder Schleimhäute, vorzugsweise der Festigkeit und/oder der Elastizität und/oder der Dichte gesunder Haut und/oder gesunder Schleimhäute, noch stärker bevorzugt der Festigkeit und/oder der Elastizität gesunder Haut und/oder gesunder Schleimhäute.

4. Verwendung nach einem der vorhergehenden Ansprüche, wobei der Stamm in ganzer, insbesondere lebensfähiger und/oder inaktivierter, insbesondere toter, Form und/oder in Lysatform und/oder in Form einer oder mehrerer von dessen Fraktionen und/oder in Form eines oder mehrerer seiner Metaboliten, vorzugsweise seines Sekretoms, verwendet wird.

5. Verwendung nach einem der vorhergehenden Ansprüche, wobei der Stamm in lebensfähiger ganzer Form und/oder in Lysatform verwendet wird und/oder wobei der verwendete Stamm der unter der Bezeichnung CNCM I-5579 hinterlegte *Lactobacillus crispatus-Stamm* ist.

6. Verwendung nach einem der vorhergehenden Ansprüche, wobei der *Lactobacillus crispatus-Stamm* mit seinem Fermentations- und/oder Kulturmedium assoziiert ist.

7. Verwendung nach einem der vorhergehenden Ansprüche, wobei der *Lactobacillus crispatus-Stamm* topisch auf gesunde Haut und/oder gesunde Schleimhäute aufgebracht wird.

8. Verwendung nach einem der vorhergehenden Ansprüche, so dass der *Lactobacillus crispatus-Stamm* in der kosmetischen Zusammensetzung in einer Konzentration von 1x10-4 Gew.-% bis 10 Gew.-%, vorzugsweise zwischen 1x10-4 Gew.-% und 5 Gew.-%, noch vorteilhafter zwischen 1x10-3 Gew.-% und 0,5 Gew.-%, bezogen auf das Gesamtgewicht der Zusammensetzung, vorhanden ist, wobei die Zusammensetzung außerdem mindestens einen kosmetisch akzeptablen Exzipienten umfasst.

9. Kosmetisches Pflegeverfahren, **dadurch gekennzeichnet, dass** es die topische Anwendung, auf mindestens einen Bereich gesunder Haut und/oder gesunder Schleimhaut, eines *Lactobacillus crispatus-Stamms* nach einem der Ansprüche 4, 5 oder 6 oder einer kosmetischen Zusammensetzung nach Anspruch 8 zum Aufrechterhalten und/oder Erhöhen der Expression von Molekülen der extrazellulären Matrix gesunder Haut und/oder gesunder Schleimhäute, vorzugsweise zum Aufrechterhalten und/oder Erhöhen der Expression der Kollagene gesunder Haut und/oder gesunder Schleimhäute und/oder der elastischen Fasern gesunder Haut und/oder gesunder Schleimhäute, und/oder zum Aufrechterhalten und/oder Erhöhen der biomechanischen Eigenschaften gesunder Haut und/oder gesunder Schleimhäute, insbesondere der Festigkeit und/oder der Elastizität und/oder der Dichte, vorzugsweise der Festigkeit, umfasst.

10. Kosmetisches Pflegeverfahren nach Anspruch 9, wobei die topische Anwendung eines *Lactobacillus crispatus-*Stamms nach einem der Ansprüche 4, 5 oder 6 oder einer kosmetischen Zusammensetzung nach Anspruch 8 auf den gesamten Körper oder einen Teil davon und/oder das Gesicht und/oder die Kopfhaut, vorzugsweise die Beine, die Oberschenkel, die Arme, den Bauch, das Dekolleté, den Hals, die Achselhöhlen, die Lippen, noch stärker bevorzugt das gesamte Gesicht oder einen Teil davon und vorzugsweise die Wangen, die Stirn, das Kinn, die Lippen, die Augenkontur durchgeführt wird.

11. Kosmetisches Pflegeverfahren nach einem der Ansprüche 9 bis 10, wobei die kosmetische Zusammensetzung den *Lactobacillus crispatus-Stamm* nach einem der Ansprüche 4, 5 oder 6 in einer Konzentration von 1x10-4 Gew.-% bis 10 Gew.-%, vorzugsweise zwischen 1x10-4 Gew.-% und 5 Gew.-%, noch vorteilhafter zwischen 1x10-3 Gew.-% und 0,5 Gew.-%, bezogen auf das Gesamtgewicht der Zusammensetzung, umfasst, wobei die Zusammensetzung außerdem mindestens einen kosmetisch akzeptablen Exzipienten umfasst.

12. *Lactobacillus crispatus-Stamm* oder diesen enthaltende dermatologische Zusammensetzung für die, vorteilhafterweise topische, Verwendung zur Behandlung und/oder Vorbeugung von Pathologien der Haut und/oder der Schleimhäute, die eine Verringerung der Menge und/oder der Expression von Molekülen der extrazellulären Matrix der Haut und/oder der Schleimhäute und/oder eine Verringerung der Menge und/oder der Expression der Kollagene und/oder der elastischen Fasern der Haut und/oder der Schleimhäute und/oder eine Verringerung der biomechanischen Eigenschaften der Haut und/oder der Schleimhäute, insbesondere eine Verringerung der Festigkeit der Haut und/oder der Schleimhäute und/oder eine Verringerung der Elastizität der Haut und/oder der Schleimhäute und/oder eine Verringerung der Dichte der Haut und/oder der Schleimhäute, mit sich bringen,
wobei der Stamm in ganzer, insbesondere lebensfähiger und/oder inaktivierter, insbesondere toter, Form und/oder in Lysatform und/oder in Form einer oder mehrerer von dessen Fraktionen und/oder in Form eines oder mehrerer seiner Metaboliten, vorzugsweise seines Sekretoms, verwendet wird
und/oder der Stamm mit seinem Fermentations- und/oder Kulturmedium assoziiert ist.

13. *Lactobacillus crispatus-Stamm* für die Verwendung nach Anspruch 12, **dadurch gekennzeichnet, dass** er in Form einer dermatologischen Zusammensetzung vorliegt, die mindestens einen dermatologisch akzeptablen Exzipienten umfasst.

14. *Lactobacillus crispatus-Stamm* für die Verwendung nach einem der Ansprüche 12 bis 13, **dadurch gekennzeichnet, dass** er in der pharmazeutischen, vorzugsweise dermatologischen, Zusammensetzung in einer Konzentration von 1x10-4 Gew.-% bis 10 Gew.-%, vorzugsweise zwischen 1x10-4 Gew.-% und 5 Gew.-%, noch vorteilhafter zwischen 1x10-3 Gew.-% und 0,5 Gew.-%, bezogen auf das Gesamtgewicht der Zusammensetzung, vorhanden ist, wobei die Zusammensetzung außerdem mindestens einen pharmazeutisch akzeptablen Exzipienten umfasst.

15. *Lactobacillus crispatus-Stamm* für die Verwendung nach einem der Ansprüche 12 bis 14, **dadurch gekennzeichnet, dass** er ein Stamm nach einem der Ansprüche 4, 5 oder 6 ist.

## Claims

1. Cosmetic and/or nutraceutical use of a strain of *Lactobacillus crispatus,* the strain of *Lactobacillus crispatus* preferentially being the species deposited under the designation CNCM I-5579, for maintaining and/or increasing the expression of extracellular matrix molecules of healthy skin and/or healthy mucous membranes.

2. Use according to Claim 1, in which the extracellular matrix molecules of healthy skin and/or healthy mucous membranes are chosen from collagen fibres of healthy skin and/or healthy mucous membranes, and/or elastic fibres of healthy skin and/or healthy mucous membranes.

3. Use according to Claim 1 or 2, for maintaining and/or increasing the expression of type I, III, IV, V, VI, VII, XII, XIII, XIV, XVI, XVII, XXIV and/or XXIX collagens, preferentially of type I, III, IV, V and/or XIV, preferentially type I, IV and/or V collagen fibres, more preferentially type I collagen fibres; and/or for maintaining and/or increasing the biomechanical properties of healthy skin and/or healthy mucous membranes, preferentially the firmness and/or elasticity and/or density of healthy skin and/or healthy mucous membranes, more preferentially the firmness and/or elasticity of healthy skin and/or healthy mucous membranes.

4. Use according to any one of the preceding claims, in which the strain is used in whole form, notably viable and/or inactivated, notably dead, and/or in the form of a lysate, and/or in the form of one or more of its fractions, and/or in the form of one or more of its metabolites, preferentially its secretome.

5. Use according to any one of the preceding claims, in which the strain is used in viable whole form and/or in lysate form and/or in which the strain used is the *Lactobacillus crispatus* strain deposited under the designation CNCM 1-5579.

6. Use according to one of the preceding claims, in which the *Lactobacillus crispatus* strain is combined with its fermentation and/or culture medium.

7. Use according to one of the preceding claims, in which the *Lactobacillus crispatus* strain is applied topically to healthy skin and/or healthy mucous membranes.

8. Use according to any one of the preceding claims, such that the *Lactobacillus crispatus* strain is present in the cosmetic composition at a concentration of 1×10⁻⁴% to 10% by weight, preferentially between 1x10⁻⁴% and 5% by weight, and more advantageously between 1×10⁻³% and 0.5% by weight, relative to the total weight of the composition, said composition also comprising at least one cosmetically acceptable excipient.

9. Cosmetic care process, **characterized in that** it comprises the topical application to at least one area of healthy skin and/or healthy mucous membrane of a strain of *Lactobacillus crispatus* according to one of Claims 4, 5 and 6, or of a cosmetic composition according to Claim 8, for maintaining and/or increasing the expression of extracellular matrix molecules of healthy skin and/or healthy mucous membranes, preferentially for maintaining and/or increasing the expression of collagens of healthy skin and/or healthy mucous membranes, and/or elastic fibres of healthy skin and/or healthy mucous membranes, and/or for maintaining and/or increasing the biomechanical properties of healthy skin and/or healthy mucous membranes, notably the firmness and/or elasticity and/or density, preferentially the firmness.

10. Cosmetic care process according to Claim 9, in which the topical application of a strain of *Lactobacillus crispatus* according to one of Claims 4, 5 and 6, or of a cosmetic composition according to Claim 8, is performed on all or part of the body and/or face and/or scalp, preferentially the legs, thighs, arms, stomach, neckline, neck, armpits or lips, more preferentially all or part of the face, and preferentially the cheeks, forehead, chin, lips or contour of the eyes.

11. Cosmetic care process according to either of Claims 9 and 10, in which the cosmetic composition comprises the strain of *Lactobacillus crispatus* according to one of Claims 4, 5 and 6 at a concentration of 1x10⁻⁴% to 10% by weight, preferentially between 1x10⁻⁴% and 5% by weight, and more advantageously between 1×10⁻³% and 0.5% by weight, relative to the total weight of the composition, said composition also comprising at least one cosmetically acceptable excipient.

12. Strain of *Lactobacillus crispatus,* or a dermatological composition comprising it, for its use, advantageously its topical use, for treating and/or preventing pathologies of the skin and/or mucous membranes involving a decrease in the amount and/or expression of extracellular matrix molecules of the skin and/or mucous membranes, and/or a decrease in the amount and/or expression of collagens and/or elastic fibres of the skin and/or mucous membranes, and/or a decrease in the biomechanical properties of the skin and/or mucous membranes, notably a decrease in the firmness of the skin and/or mucous membranes, and/or a decrease in the elasticity of the skin and/or mucous membranes, and/or a decrease in the density of the skin and/or mucous membranes,
the strain being used in whole form, notably viable and/or inactivated, notably dead, and/or in the form of a lysate, and/or in the form of one or more of its fractions, and/or in the form of one or more of its metabolites, preferentially its secretome,
and/or the strain being combined with its fermentation and/or culture medium.

13. Strain of *Lactobacillus crispatus* for its use according to Claim 12, **characterized in that** it is in the form of a dermatological composition comprising at least one dermatologically acceptable excipient.

14. Strain of *Lactobacillus crispatus* for its use according to either of Claims 12 and 13, **characterized in that** it is present in the pharmaceutical, preferentially dermatological, composition at a concentration of 1x10⁻⁴% to 10% by weight, preferentially between 1x10⁻⁴% and 5% by weight, more advantageously between 1×10⁻³% and 0.5% by weight relative to the total weight of the composition, said composition also comprising at least one pharmaceutically acceptable excipient.

15. Strain of *Lactobacillus crispatus* for its use according to any one of Claims 12 to 14, **characterized in that** the strain is according to one of Claims 4, 5 and 6.
